# FASCICULE DE BREVET EUROPEEN

(11) **EP 1 563 071 B1**
(45) Date de publication et mention de la délivrance du brevet: **17.09.2008**
(21) Numéro de dépôt: 03786019.4
(22) Date de dépôt: 18.11.2003
(51) Int. Cl.: C12N 15/12, C07K 14/705, C07K 16/28, C12Q 1/68, G01N 33/50, G01N 33/53, A01K 67/027, C12N 15/11

(54) **PROTEINE SPECIFIQUE DES CELLULES PANCREATIQUES BETA DES ILOTS DE LANGERHANS ET SES APPLICATIONS.**
PROTEIN SPEZIFISCH FÜR DIE PANKREATISCHEN BETA-ZELLEN DER LANGERHANS-INSELN, UND SEINE VERWENDUNGEN
PROTEIN SPECIFIC TO PANCREATIC BETA CELLS IN ISLETS OF LANGERHANS AND APPLICATIONS THEREOF

(30) Priorité: 18.11.2002 FR 0214374
(43) Date de publication de la demande: 17.08.2005
(60) Demande divisionnaire: 08002520.8
(73) Titulaire: COMMISSARIAT A L'ENERGIE ATOMIQUE, 75015 Paris (FR); UNIVERSITE JOSEPH FOURIER (GRENOBLE 1), F-38041 Grenoble Cédex (FR)
(72) Inventeur: SEVE, Michel, F-38000 Grenoble (FR); FAVIER, Alain, F-38190 Bernin (FR)
(74) Mandataire: Marcadé, Véronique
(86) Numéro de dépôt international: PCT/FR2003/003413
(87) Numéro de publication internationale: WO 2004/046355

(56) Documents cités:
- WO-A-01/42789
- WO-A-02/24733
- DATABASE EMBL [en ligne] ik02a02.y1 Human insulinoma, 9 mai 2002 (2002-05-09) MELTON D. ET AL.: "Homo sapiens cDNA clone IMAGE: 5779491 5' similar to TR:Q62941 zinc transporter ZNT-2; mRNA sequence" retrieved from EBI Database accession no. BQ267316 XP002246606
- DATABASE EMBL [en ligne] ik36b01.y1 HR85 islet, MELTON D. ET AL.: "Homo sapiens cDNA clone IMAGE: 5782969 5' similar to TR:Q62941 zinc transporter ZNT-2; mRNA sequence" retrieved from EBI Database accession no. BQ417284 XP002246607
- DATABASE EMBL [en ligne] in61h03.y1 HR85 islet, MELTON D. ET AL.: "Homo sapiens cDNA clone IMAGE: 5782969 5' similar to TR:Q62941 Zinc transporter ZNT-2; mRNA sequence" retrieved from EBI Database accession no. BU949895 XP002246608
- TOMITA T: "New markers for pancreatic islets and islet cell tumors" PATHOLOGY INTERNATIONAL 2002 JAPAN, vol. 52, no. 7, 2002, pages 425-432, XP002246605 ISSN: 1320-5463

## Description

La présente Invention est relative à une protéine dénommée ZnT-8, exprimée de manière spécifique dans les cellules pancréatiques bêta des îlots de Langerhans, au polynucléotide codant ladite protéine qui est impliquée dans la maturation et l'exocytose de l'insuline, ainsi qu'à leurs applications notamment pour le tri et l'étude des cellules bêta, ainsi que pour le criblage de médicaments actifs sur le diabète et l'hyperinsulinisme.

Le diabète est une des maladies les plus fréquentes, affectant 5 % de la population des pays industrialisés et en constante augmentation dans tous les pays du monde (prévision : 300 millions en 2025, dont 2,4 millions en France). Parmi les diverses formes de diabète, le diabète de type I ou insulino-dépendant affecte environ 500 000 à 1 million d'individus aux Etats-Unis et 150 000 en France soit 0,2 à 0,4 % de la population. Les symptômes caractéristiques comprennent un taux élevé de sucre dans le sang et dans les urines, une diurèse importante, une faim et une soif intenses ainsi qu'une perte de poids.

Le diabète de type II non insulino-dépendant (DNID), aussi décrit sous le nom de diabète "gras" ou diabète de la maturité, survient souvent autour de la cinquantaine. Il est traité par un régime, la prise de médicaments par voie orale, et l'insuline, après quelques années d'évolution. Aujourd'hui, 2 millions de Français sont traités avec des médicaments antidiabétiques et/ou de l'insuline.

Bien que le diabète puisse être contrôlé par des injections d'insuline et des apports contrôlés en glucides, les complications associées à cette pathologie nécessitent de nos jours de nouvelles approches, dans sa prévention, son traitement et son diagnostic.

Le pancréas comprend deux structures distinctes aussi bien morphologiquement que physiologiquement :
- le pancréas exocrine qui produit les enzymes intervenant dans la digestion (amylase, lipase, etc.) et le bicarbonate de sodium ;
- le pancréas endocrine qui produit les hormones intervenant dans le contrôle du glucose sanguin (insuline, glucagon, somatostatine et polypeptide pancréatique).

Les cellules du pancréas endocrine sont organisées en micro-organes dispersés dans le pancréas sous forme d'îlots (îlots de Langerhans ou îlots pancréatiques). Chaque îlot pancréatique est composé de 4 types cellulaires : les cellules alpha, bêta, delta, et les cellules PP. Les cellules alpha sont situées à la périphérie de l'îlot et secrètent le glucagon. Les cellules bêta sont retrouvées au centre de l'îlot et sont les seules cellules capables de secréter l'insuline en réponse au glucose. Les cellules delta sont à la périphérie et secrètent la somatostatine. La fonction des cellules PP est plus controversée (synthèse du polypeptide pancréatique).

L'absence de modèle cellulaire d'étude de la cellule bêta, ainsi que l'absence de moyen fiable et efficace de tri cellulaire adapté à ce type de cellule, freine l'étude de son fonctionnement et donc la mise au point de nouvelles méthodes de traitement du diabète de type I et II.

Parmi les traitements du diabète, outre l'administration régulière d'insuline, une des voies du contrôle physiologique de la glycémie et de la normalisation de la glycémie chez les diabétiques est le passage par la restauration d'une sécrétion d'insuline *in vivo* à partir de cellules. Dans cette optique plusieurs solutions ont été proposées :
- l'obtention de cellules productrices d'insuline chez l'animal pour la réalisation d'une xénotransplantation ;
- la différenciation *in vitro* en cellules sécrétrices d'insuline à partir de cellules souches isolées, dans un but de réimplantation [1], ceci afin de contourner les problèmes d'immunité et la nécessité d'un traitement immunosuppresseur du patient. Mais la production de quantités importantes de cellules à faible coût, productrices d'insuline par différenciation de cellules souches, nécessite de nouveaux outils biomoléculaires, notamment utiles pour le phénotypage et la purification des cellules différenciées ;
- la transplantation d'îlots pancréatiques ; de nombreux travaux ont eu récemment pour objet la préparation d'îlots ou de cellules bêta pancréatiques à des fins thérapeutiques. La première étape de la transplantation est le prélèvement du pancréas sur un donneur en état de mort cérébrale. L'isolement des îlots commence par une digestion enzymatique du pancréas au moyen d'une solution de collagénase. Toutes les transplantations ne requièrent pas une purification des îlots digérés. Toutefois, la plupart des chercheurs s'accordent aujourd'hui pour dire que la purification des îlots est nécessaire pour une allotransplantation [2]. Les îlots sont ensuite transplantés, à raison d'une masse suffisante (minimum 3000 IEQ/kg) par injection intraportale (IEQ : Islet Equivalent, Equivalent Ilôt).

Toutefois, l'isolement d'îlots ou de cellules bêta pancréatiques nécessite des moyens de sélection et d'identification des cellules bêta, spécifiques et fiables.

Des travaux antérieurs ont tenté de mettre au point des méthodes de marquage des cellules bêta. On peut citer :
- le marquage par la GFP (Green Fluorescent Protein) qui permet un marquage fluorescent de la cellule. L'inconvénient majeur de cette technique est la nécessité d'introduire un gène exogène ou transgène dans la cellule, qui plus est par l'utilisation d'un vecteur viral (adénovirus) [1] ;
- la technique basée sur l'autofluorescence importante de la cellule bêta [2]. Mais cette technique manque de spécificité vis-à-vis du type cellulaire ;
- l'incubation des cellules avec un fluorochrome spécifique du zinc : le Newport Green [3] ou la dithizone [4]. Cette technique est basée sur la teneur importante en zinc de la cellule bêta. Le zinc est un constituant important des granules de sécrétion de l'insuline, et en outre, joue un rôle dans le contrôle de cette sécrétion [5]. Mais ces techniques présentent de nombreux inconvénients : utilisation d'un produit chimique, risque de toxicité vis-à-vis de la cellule bêta, manque de spécificité vis-à-vis du type cellulaire. De plus, la dithizone pose un problème de photodégradation rapide [6] ;
- la mise en évidence de manière indirecte par reconnaissance par un clone de cellules T (WO 91/17186) d'un antigène exprimé par les cellules bêta. Les travaux initiaux sur cet antigène n'apportaient pas de résultats en terme de caractérisation de la séquence peptidique, ainsi qu'en terme de sélectivité de la cellule bêta par rapport aux autres types cellulaires de l'îlot, mais également du pancréas ou de l'organisme. Des travaux plus récents des mêmes auteurs montrent une distribution beaucoup plus large de cet antigène, qui de fait n'est pas spécifique de la cellule bêta [7] ;
- la détection du transporteur du glucose GLUT-2 [15] ; mais ce transporteur n'est pas spécifique des cellules bêta.

En conséquence, il existe un manque de marqueur spécifique et fiable de la cellule bêta des îlots pancréatiques de Langerhans.

C'est un des buts de la présente Invention que de fournir un tel marqueur.

L'îlot de Langerhans accumule de très grandes quantités de zinc et nécessite donc un transporteur très efficace et très spécialisé pour accumuler ce zinc dans les vésicules de sécrétion [8]. L'insuline, produite et stockée dans la cellule pancréatique bêta, est libérée dans le milieu extracellulaire par exocytose en réponse à des stimuli externes, comme une élévation de la concentration en glucose. Cette élévation du glucose provoque une modification du rapport ATP/ADP, la fermeture des canaux potassiques et l'ouverture des canaux calciques qui provoque l'exocytose [9].

Il est connu qu'en présence de zinc, l'insuline peut former des tétramères et des hexamères qui fixent le zinc dans un rapport insuline : zinc de 4 :1 et 6 :2 respectivement. L'insuline est stockée dans les granules de sécrétion sous la forme d'un solide composé d'hexamères liés à 2 atomes de zinc par hexamère. Les vésicules contiennent du zinc en un excès de 1 à 1,5 fois la quantité nécessaire pour former les hexamères insuline-zinc. Au cours de l'exocytose de l'insuline, les vésicules riches en insuline fusionnent avec la membrane plasmique de la cellule bêta et libèrent l'insuline mais également le zinc, dans la circulation. Le zinc libéré agit dans une boucle de rétrocontrôle négatif sur les canaux potassiques, provoquant son activation et l'arrêt de l'exocytose.

Dans les cellules de mammifères, 7 protéines homologues ayant une fonction de transporteur du zinc, dénommées ZnT-1, -2, -3, -4, -5, -6 et -7, ont été clonées et caractérisées. L'analyse de la structure primaire de ces protéines a permis de définir un motif structural commun composé de 6 domaines transmembranaires et d'une boucle intracellulaire riche en histidine. ZnT-1 est un transporteur ubiquitaire localisé dans la membrane plasmique qui assure l'efflux de zinc hors de la cellule [11]. ZnT-2 permet à la cellule de tolérer un excès de zinc dans le milieu de culture, conférant ainsi une résistance au zinc par une localisation de celui-ci dans des vésicules intracyto-plasmiques acides, assurant ainsi une accumulation du zinc dans la cellule bien supérieure à la normale [12]. ZnT-3 et ZnT-4 clonés chez l'homme, ont des fonctions similaires à ZnT-2. ZnT-3 est spécifique de certains tissus et fortement exprimée dans le cerveau, dans les membranes des vésicules synaptiques riches en zinc, dans les fibres moussues de l'hippocampe et dans les testicules. ZnT-4 est exprimée de manière ubiquitaire, mais des niveaux plus élevés sont retrouvés dans le cerveau et les cellules épithéliales. Ce transporteur est essentiel dans l'épithélium mammaire où il participe au contrôle du contenu en zinc du lait maternel. ZnT-5 et ZnT-6 sont aussi des transporteurs ubiquitaires localisés dans l'appareil de Golgi. ZnT-7 est un transporteur ubiquitaire, localisé spécifiquement dans le réticulum endoplasmique des cellules.

Un polynucléotide codant un polypeptide transporteur de Zinc appelé NOV2 a également été décrit dans la Demande Internationale WO 02/24733. Ledit polypeptide est exprimé au niveau non seulement du pancréas mais aussi de la moelle osseuse, du cartilage, du placenta et du rein. Par conséquent, du fait de son expression dans plusieurs tissus, ce polypeptide ne peut pas être utilisé en tant que marqueur spécifique des cellules bêta-pancréatiques.

Des travaux précédents mentionnent une tentative de recherche de gènes impliqués dans le métabolisme du zinc dans la cellule bêta pancréatique. Ces travaux n'ont pas abouti à la mise en évidence d'une protéine ou d'un transporteur spécifique [10].

Les Inventeurs ont isolé un polynucléotide de 1110 paires de bases (SEQ ID NO:1) représentant l'ADNc correspondant à un ARNm exprimé de manière spécifique dans l'îlot de Langerhans, et plus particulièrement dans la cellule sécrétrice d'insuline ou cellule bêta.

Le polynucléotide de l'Invention, code pour une protéine dénommée ZnT-8, (SEQ ID NO:2), laquelle protéine de 369 acides aminés correspondant à une masse moléculaire estimée à 40,8 KDa, présente une structure primaire homologue à celle des protéines de la famille ZnT. Le gène codant ladite protéine est dénommé *ZnT-8*.

L'étude du potentiel transmembranaire de la protéine ZnT-8 complète montre qu'elle possède 6 domaines transmembranaires (acides aminés 74-95, 107-126, 141-163, 177-196, 216-240, 246-267), les extrémités N- et C-terminales étant situées dans le cytoplasme. Cette étude montre également que la structure secondaire de cette protéine présente 3 boucles d'acides aminés extracellulaires (acides aminés 96-106, 164-176, 241-245).

En outre, la localisation de la protéine ZnT-8 dans les vésicules de sécrétion de l'Insuline et sur la membrane plasmique indiquent qu'elle est impliquée dans l'accumulation du zinc dans les vésicules contenant l'insuline et joue donc un rôle dans la maturation et l'exocytose de l'insuline dans les cellules bêta des îlots pancréatiques de Langerhans.

La protéine ZnT-8 et le polynucléotide correspondant constituent pour la première fois un marqueur spécifique et fiable de la cellule bêta des îlots pancréatiques de Langerhans ; les utilisations de ce marqueur sont notamment les suivantes :
- tri cellulaire ; le marqueur permet d'envisager un tri et un repérage sélectif des cellules bêta, sans modification chimique ou biologique desdites cellules, notamment à l'aide d'anticorps dirigés contre ladite protéine.
- modèle d'étude *in vitro* ; le marqueur peut être utilisé *in vitro* pour étudier : (i) la surexpression du transporteur (ZnT-8) dans des lignées de cellules modèles (insulinome de rat INS-1 par exemple) et de l'impact sur la sécrétion de l'insuline en réponse à une stimulation par le glucose, (ii) la sensibilité des cellules à la mort cellulaire (apoptose) induite par des conditions de stress oxydant ou de concentration faible ou forte en zinc, et (iii) les étapes de différenciation de cellules souches en cellules sécrétrices d'insuline en réponse à différentes stimulations exogène (facteurs de croissance, extraits pancréatiques).
- criblage de médicaments : le marqueur représente également une cible pharmacologique utile pour le criblage de substances capables de moduler l'expression du gène *ZnT*-*8* et/ou l'activité de la protéine ZnT-8, potentiellement utilisables pour le traitement du diabète et de l'hyperinsulinisme.
- diagnostic du diabète : le polynucléotide peut également avantageusement être mis en oeuvre dans le diagnostic précoce du diabète dans les familles à risque, notamment en ce qu'il permet de détecter des mutations observables dans le gène *ZnT*-*8* et aussi de diminuer voir de supprimer les examens habituellement mis en oeuvre.

Ainsi, la présente Invention a pour objet l'utilisation en tant que marqueur spécifique des cellules bêta des îlots pancréatiques de Langerhans d'au moins un polynucléotide isolé ou de la protéine correspondante choisis parmi :
- les polynucléotides comprenant ou présentant l'une des séquences suivantes : (a) la séquence SEQ ID NO:1, (b) un fragment de la séquence SEQ ID NO:1 d'au moins 15 nucléotides consécutifs, de préférence 20 nucléotides, encore plus préférentiellement 25 à 30 nucléotides, (c) une séquence présentant un pourcentage d'identité d'au moins 80 % après alignement optimal avec l'une des séquences définies en (a) ou en (b), et (d) une séquence complémentaire, sens ou antisens de l'une des séquences définies en (a), (b) ou (c), et
- les protéines codées par les polynucléotides tels que définis en (a), (b), (c) ou (d) ci-dessus comprenant ou présentant l'une des séquences suivantes : (e) la séquence SEQ ID NO:2, (f) un fragment de la séquence SEQ ID NO:2 d'au moins 15 acides aminés consécutifs, (g) une séquence présentant un pourcentage d'identité d'au moins 60 % après alignement optimal avec l'une des séquences définies en (e) ou en (f) ou au moins 65 % de similarité, de préférence 80 % d'identité ou au moins 90 % de similarité, ou de manière encore plus préférée 90 % d'identité ou au moins 95 % de similarité.

L'invention englobe les utilisations de la protéine ZnT-8 et du polynucléotide correspondant telles que définies ci-dessus.

Le polynucléotide tel que défini ci-dessus, peut être isolé à partir de cellules de Langerhans ou à partir de banques d'ADN cellulaire, particulièrement de banques d'ADN de cellules pancréatiques, très particulièrement à partir d'une banque d'ADN de cellules pancréatiques humaines. De préférence, les cellules utilisées sont des cellules des îlots de Langerhans.

Le polynucléotide tel que défini ci-dessus peut également être obtenu par une réaction de polymérisation en chaîne (PCR) effectuée sur l'ADN total des cellules de Langerhans, par RT-PCR effectuée sur les ARN totaux des cellules bêta d'îlots pancréatiques de Langerhans ou par synthèse chimique.

Au sens de la présente Invention les définitions suivantes s'appliquent.

Par polynucléotide, on entend un enchaînement précis de nucléotides, modifiés ou non, comportant ou non des nucléotides non naturels. Ainsi, ce terme recouvre toute séquence qui code pour une protéine ZnT-8 ou un fragment de ladite protéine (ADN génomique, ARNm, ADNc) mais aussi les oligonucléotides, sens ou antisens, ainsi que les petits ARN interférents (small interfering RNA, siRNA) correspondants.

Par "acides nucléiques ou protéines présentant un pourcentage d'identité après alignement optimal avec une séquence de référence", on entend désigner les acides nucléiques ou les protéines présentant, par rapport à la séquence de référence, certaines modifications comme en particulier une délétion, une troncation, un allongement, une fusion chimérique, et/ou une substitution, notamment ponctuelle, et dont la séquence en nucléotides présente au moins 80 % d'identité et la séquence en acides aminés présente au moins 65 % d'identité après alignement optimal avec la séquence en nucléotides ou en acides aminés de référence.

Par "pourcentage d'identité" entre deux séquences (acides nucléiques ou protéines), on entend désigner un pourcentage de nucléotides ou de résidus d'acides aminés identiques entre les deux séquences à comparer, obtenu après le meilleur alignement, ce pourcentage étant purement statistique et les différences entre les deux séquences étant réparties au hasard et sur toute leur longueur.

Par "meilleur alignement" ou "alignement optimal", on entend désigner l'alignement pour lequel le pourcentage d'identité déterminé comme décrit ci-après est le plus élevé. Les comparaisons entre deux séquences de nucléotides ou d'acides aminés sont traditionnellement réalisées : en comparant ces séquences après les avoir alignées de manière optimale, ladite comparaison étant réalisée par segment ou par "fenêtre de comparaison" pour identifier et comparer les régions locales de similarité de séquence. L'alignement optimal des séquences pour la comparaison peut être réalisé, notamment à l'aide de l'un des algorithmes suivants : l'algorithme d'homologie locale de Smith et Waterman (1981), l'algorithme d'homologie locale de Neddleman et Wunsch (1970), la méthode de recherche de similarité de Pearson et Lipman (1988), les logiciels informatiques utilisant ces algorithmes (GAP, BESTFIT, BLAST P, BLAST N, BLASTX, TBLASTX, FASTA et TFASTA dans le Wisconsin Genetics Software Package, (Genetics Computer Group, 575 Science Dr., Madison, WI) ou sur les serveurs internet, en particulier ceux du *National Center for Biotechnology Information* (http ://www.ncbi.nlm.nih.gov), de l'EMBL (http ://www.embl.org) et du projet Ensembl (http ://www.ensembl.org)).

Afin d'obtenir l'alignement optimal, on utilise de préférence le programme BLAST, avec la matrice BLOSUM 62. On peut également utiliser les matrices PAM ou PAM250, ainsi qu'une matrice d'identité pour les séquences de nucléotides.

Pour obtenir une "hybridation spécifique" on utilise de préférence des conditions d'hybridation de forte stringence, c'est-à-dire des conditions de température et de force ionique choisies de telle manière qu'elles permettent le maintien de l'hybridation spécifique et sélective entre polynucléotides complémentaires.

A titre d'illustration, des conditions de forte stringence de l'étape d'hybridation aux fins de définir les polynucléotides décrits ci-dessus, sont avantageusement les suivantes : l'hybridation ADN-ADN ou ADN-ARN est réalisée en deux étapes : (1) préhybridation à 42°C pendant 3 heures en tampon phosphate (20 mM pH 7,5) contenant 5 x SSC (1 x SSC correspond à une solution 0,15 M NaCl + 0, 015 M citrate de sodium), 50 % de formamide, 7 % de sodium dodécyl sulfate (SDS), 10 x Denhardt's, 5 % de dextran sulfate et 1 % d'ADN de sperme de saumon ; (2) hybridation proprement dite pendant 20 heures à une température dépendant de la taille de la sonde (i.e. : 42°C, pour une sonde de taille supérieure à 100 nucléotides) suivie de 2 lavages de 20 minutes à 20°C en 2 x SSC + 2 % SDS, 1 lavage de 20 minutes à 20°C en 0,1 x SSC + 0,1 % SDS. Le dernier lavage est pratiqué en 0,1 x SSC + 0,1 % SDS pendant 30 minutes à 60°C pour une sonde de taille supérieure à 100 nucléotides. Les conditions d'hybridation de forte stringence décrites ci-dessus pour une sonde de taille définie peuvent être adaptées par l'Homme du Métier pour des sondes de taille plus grande ou plus petite.

Par "techniques ou méthodes appropriées" on entend ici se référer aux techniques ou méthodes bien connues et classiquement utilisées par l'Homme du Métier et exposées dans de nombreux ouvrages, comme en particulier celui intitulé Molecular Cloning. A Laboratory Manual (Sambrook J, Russell DW. (2000) Cold Spring Harbor Laboratory Press) [13].

Le polynucléotide tel que défini en c) présente un pourcentage d'identité d'au moins 80 % après alignement optimal avec une séquence telle que définie en a) ou b), de préférence 90 %, de façon plus préférée 95 %, de façon encore plus préférée 98 %. Le polynucléotide tel que défini en c) inclut les polynucléotides variants de la séquence SEQ ID NO:1, c'est-à-dire l'ensemble des polynucléotides correspondants à des variants alléliques, c'est-à-dire à des variations individuelles de la séquence SEQ ID NO:1. Ces séquences variantes naturelles correspondent à des polymorphismes présents chez les mammifères, en particulier chez l'être humain et, notamment à des polymorphismes pouvant conduire à la survenue d'une pathologie, comme par exemple la mort cellulaire des îlots de Langerhans et un diabète.

On entend également désigner par polynucléotide variant, tout ARN ou ADNc résultant d'une mutation et/ou d'une variation d'un site d'épissage de la séquence génomique dont l'ARNm a comme ADN complémentaire le polynucléotide de séquence SEQ ID NO:1.

La similarité d'une protéine par rapport à une protéine de référence s'apprécie en fonction du pourcentage de résidus d'acides aminés qui sont identiques ou qui différent par des substitutions conservatives, lorsque les deux séquences sont alignées de manière à obtenir le maximum de correspondance entre elles. Au sens de la présente Invention, on entend par substitution conservative, la substitution d'un acide aminé par un autre qui présente des propriétés chimiques similaires (taille, charge ou polarité), qui généralement ne modifie pas les propriétés fonctionnelles de la protéine.

Une protéine ayant une séquence en acides aminés ayant au moins X % de similarité avec une séquence de référence est définie, dans la présente Invention comme une protéine dont la séquence peut inclure jusqu'à 100-X altérations non-conservatives pour 100 acides aminés de la séquence de référence. Au sens de la présente Invention, le terme altérations non-conservatives inclut les délétions, les substitutions non-conservatives ou les insertions consécutives ou dispersées d'acides aminés dans la séquence de référence.

Sont incluses dans les protéines telles que définies en (g) les protéines variantes de la séquence SEQ ID NO:2, c'est-à-dire les protéines variantes codées par les polynucléotides variants tels que précédemment définis, en particulier les protéines dont la séquence en acides aminés présente au moins une mutation correspondant notamment à une troncation, une délétion, une substitution et/ou une addition d'au moins un résidu d'acide aminé par rapport à la séquence SEQ ID NO:2.

De manière préférée, les protéines variantes présentent une mutation associée au diabète ou à l'hyperinsulinisme.

Selon un mode de réalisation avantageux de l'utilisation selon la présente invention, ledit polynucléotide isolé tel que défini en (c), est un polynucléotide variant de la séquence SEQ ID NO:1 comportant une mutation qui conduit à une modification de la séquence en acides aminés de la protéine codée par la séquence SEQ ID NO:1.

Selon un autre mode de réalisation avantageux de l'utilisation selon la présente invention, lesdits polynucléotides isolés tel que définis en (b) ou en (d) sont choisis parmi la paires d'amorces SEQ ID NO:3 et SEQ ID NO:27 (séquence complémentaire de la SEQ ID NO:4) et la paire d'amorce SEQ ID NO:5 et SEQ ID NO:6.

Selon encore un autre mode de réalisation avantageux de l'utilisation selon la présente invention, ledit polynucléotide isolé est susceptible d'être obtenu par amplification à l'aide de la paire d'amorces telle que définie ci-dessus.

Selon encore un autre mode de réalisation avantageux de l'utilisation selon la présente invention, ledit polynucléotide tel que défini en (d) est un petit ARN interférent (siRNA) qui par interaction avec les ARNm correspondants audit polynucléotide, conduira à leur dégradation.

Selon encore un autre mode de réalisation avantageux de l'utilisation selon la présente invention, ladite protéine telle que définie en (g) est un variant de la séquence SEQ ID NO:2 présentant une mutation associée au diabète ou à l'hyperinsulinisme.

Selon encore un autre mode de réalisation avantageux de l'utilisation selon la présente invention, ledit fragment tel que défini en (f) présente une séquence choisie parmi les séquences SEQ ID NO:7, SEQ ID NO:8, SEQ ID NO:9 et SEQ ID NO:10.

La présente invention a également pour objet un polynucléotide tel que défini ci-dessus, à l'exception :
- des fragments d'au moins 15 nucléotides consécutifs inclus dans les séquences présentant les numéros d'accès dans la base de données NCBI n° AX526723 (SEQ ID NO:11), n° AX526725 (SEQ ID NO:12) et n° AX526727 (SEQ ID NO:13),
- des EST présentant les numéros d'accès dans la base de données GenBank BM565129 (SEQ ID NO:14), BM310003 (SEQ ID NO:15), BM875526 (SEQ ID NO:16), BG655918 (SEQ ID NO:17), BQ417284 (SEQ ID NO:18), BQ267316 (SEQ ID NO:19), BU072134 (SEQ ID NO:20), BQ267526 (SEQ ID NO:21), BQ270198 (SEQ ID NO:22), BU581447 (SEQ ID NO:23), BU070173 (SEQ ID NO:24), BQ631692 (SEQ ID NO:25) et BU949895 (SEQ ID NO:26), ainsi que des séquences présentant les numéros d'accès dans la base de données NCBI AX526723 (SEQ ID NO:11), AX526725 (SEQ ID NO:12) et AX526727 (SEQ ID NO:13).

Les fragments selon l'Invention peuvent notamment être utilisés comme sondes ou comme amorces pour détecter/amplifier des polynucléotides (ARN ou ADN génomique) correspondants au polynucléotide selon l'Invention dans d'autres organismes.

Préférentiellement les paires d'amorces utilisables selon l'Invention sont celles correspondants aux paires définies par les séquences SEQ ID NO:3 et SEQ ID NO:4 et par les séquences SEQ ID NO:5 et SEQ ID NO:6.

L'Invention a également pour objet les polynucléotides susceptibles d'être obtenus par amplification à l'aide des amorces telles que définies ci-dessus.

Selon un mode de réalisation avantageux du polynucléotide selon l'invention, il s'agit d'un petit ARN interférent correspondant au polynucléotide tel que défini ci-dessus, d'une taille inférieure à 30 nucléotides, de préférence d'une taille comprise entre 20 et 23 nucléotides, qui par interaction avec les ARNm correspondants audit polynucléotide, conduira à leur dégradation. Ces siRNA peuvent être obtenus par toute méthode connue de l'Homme du Métier, par exemple par synthèse chimique ou encore par expression à partir d'un vecteur.

L'Invention englobe les oligonucléotides sens correspondants aux polynucléotides de l'Invention qui, par interaction avec des protéines impliquées dans la régulation de l'expression dudit polynucléotide de l'Invention, induiront soit une inhibition, soit une activation de cette expression.

Les sondes et amorces selon l'Invention peuvent être marquées directement ou indirectement par un composé radioactif ou non radioactif par des méthodes bien connues de l'Homme du Métier, afin d'obtenir un signal détectable et/ou quantifiable.

Le marquage des amorces ou des sondes selon l'Invention est réalisé par des éléments radioactifs ou par des molécules non radioactives. Parmi les isotopes radioactifs utilisés, on peut citer le ³²P, le ³³P, le ³⁵S, le ³H ou l'¹²⁵I. Les entités non radioactives sont sélectionnées parmi les ligands tels que la biotine, l'avidine, la streptavidine, la digoxygénine, les haptènes, les colorants, les agents luminescents tels que les agents radioluminescents, chémoluminescents, bioluminescents, fluorescents, phosphorescents.

Les polynucléotides selon l'Invention peuvent ainsi être utilisés comme amorce et/ou sonde dans des procédés mettant en oeuvre notamment la technique de PCR (amplification en chaîne par polymérase) (U.S. N° 4,683,202). D'autres techniques d'amplification de l'acide nucléique cible peuvent être avantageusement employées comme alternative à la PCR. Il existe actuellement de très nombreux procédés permettant cette amplification, comme par exemple la technique SDA (Strand Displacement Amplification) ou technique d'amplification à déplacement de brin, la technique TAS (Transcription-based Amplification System), la technique 3SR (Self-Sustained Sequence Replication), la technique NASBA (Nucleic Acid Sequence Based Amplification), la technique TMA (Transcription Mediated Amplification), la technique LCR (Ligase Chain Reaction), la technique de RCR (Repair Chain Reaction), la technique CPR (Cycling Probe Reaction), la technique d'amplification à la Q-bêta-réplicase. On peut encore citer la PCR-SSCP qui permet de détecter des mutations ponctuelles.

Ces techniques sont bien entendu parfaitement connues de l'Homme du Métier.

Comme sondes ou comme amorces, les différents polynucléotides de l'Invention permettent, soit de déterminer le profil de transcription du gène correspondant ou une éventuelle altération de ce profil dans un échantillon biologique, soit de mettre en évidence le gène correspondant, des variants alléliques de ce gène ou une éventuelle altération fonctionnelle de ce gène (changement substantiel dans l'activité de la protéine codée par ledit gène) résultant d'une mutation (insertion, délétion ou substitution) d'un ou plusieurs nucléotides au niveau d'au moins un exon dudit gène. De telles mutations incluent en particulier les délétions, les insertions ou les substitutions non-conservatives au niveau de codons correspondant à des résidus d'acides aminés situés dans un domaine essentiel pour l'activité biologique de la protéine.

Ainsi l'Invention a pour objet une méthode de détermination du profil de transcription du gène correspondant au polynucléotide de l'Invention ou d'une altération dudit profil, dans un échantillon biologique, comprenant une première étape d'obtention des ARN totaux à partir de l'échantillon biologique, une deuxième étape de mise en contact desdits ARN avec une sonde marquée constituée d'un polynucléotide selon l'Invention dans des conditions appropriées d'hybridation entre les ARN et la sonde et une troisième étape de révélation par tout moyen approprié des hybrides formés.

Selon un mode de mise en oeuvre de ladite méthode, la deuxième étape peut être une étape de rétrotranscription et/ou d'amplification des transcrits réalisée à l'aide d'une paire d'amorces telle que décrite précédemment et la troisième étape, une étape de révélation par tout moyen approprié des acides nucléiques amplifiés formés.

Ladite méthode de détermination du profil de transcription du gène peut en outre comporter une étape d'évaluation du taux de transcription du gène par comparaison avec un échantillon témoin préalablement choisi et éventuellement l'étude de sa corrélation avec un phénotype détectable comme par exemple le taux de proinsuline convertie en insuline mature, le contenu des cellules en insuline, le taux d'insuline secrétée en réponse à une stimulation par le glucose, la concentration intracellulaire ou intravésiculaire en zinc ou encore la quantité de protéine (produit du gène) exprimée à la surface de la cellule). Ledit échantillon témoin peut être constitué par exemple par un échantillon biologique présentant une transcription normale ou altérée du gène correspondant au polynucléotide de l'Invention auquel ladite méthode de détermination du profil de transcription du gène est appliquée dans les mêmes conditions.

L'Invention a aussi pour objet une méthode de mise en évidence du gène correspondant au polynucléotide de l'Invention ou des variants alléliques dudit gène ou d'une altération fonctionnelle de ce gène, dans un échantillon biologique, comprenant une première étape d'obtention par tout moyen approprié de l'ADN à partir de l'échantillon biologique, une deuxième étape de mise en contact desdits ADN avec une sonde marquée constituée d'un polynucléotide selon l'Invention dans des conditions appropriées d'hybridation spécifique entre les ADN et la sonde et une troisième étape de révélation par tout moyen approprié des hybrides formés.

Selon un mode de mise en oeuvre avantageux de ladite méthode la deuxième étape peut être une étape d'amplification réalisée à l'aide d'une paire d'amorces telle que décrite précédemment et la troisième étape, une étape de révélation par tout moyen approprié des acides nucléiques amplifiés formés. La méthode peut éventuellement comporter une quatrième étape d'isolement et de séquençage des acides nucléiques mis en évidence.

Cette dernière méthode peut également permettre d'isoler un allèle du gène correspondant au polynucléotide de l'Invention, associé à un phénotype détectable, comme par exemple une variation de la glycémie post-prandiale, la présence ou non d'une sécrétion d'insuline, le taux de glucose circulant ou d'insuline secrétée en réponse à une stimulation par du glucose, et d'une manière générale à une pathologie de type diabète de type I ou II ou encore à une anomalie du métabolisme du zinc. Le zinc excrété au cours de la libération de l'insuline agit sur les canaux potassiques, responsables par l'intermédiaire de canaux calciques de cette exocytose. Il y a donc une boucle de rétrocontrôle [14]. Le polypeptide de l'invention est impliqué dans l'accumulation du zinc dans les vésicules contenant l'insuline, et se retrouve également au cours de l'exocytose sur la membrane plasmique. Des mutants de la protéines pourraient donc modifier soit la quantité de zinc présent dans les vésicules, soit la concentration péricellulaire en zinc, et donc modifier l'état d'ouverture du canal potassique, aboutissant suivant l'effet de la mutation à une diminution ou une augmentation de l'excrétion d'insuline (diabète de type I ou hyperinsulinisme). Dans cette méthode particulière, l'échantillon biologique sera un échantillon provenant d'un individu exprimant ledit phénotype détectable.

Ces méthodes, particulièrement celles basées sur la recherche de mutations dans le gène, peuvent permettre la mise en évidence de façon préventive d'une prédisposition au diabète, ou le diagnostic du diabète ou de toute maladie liée au diabète, ou l'adaptation en terme de molécule ou de posologie du ou des traitements antidiabétiques.

L'Invention a également pour objet une trousse de réactifs pour la mise en oeuvre des méthodes précédemment décrites comprenant :
a) au moins une sonde ou une paire d'amorces selon l'Invention ;
b) les réactifs nécessaires à la mise en oeuvre d'une réaction d'hybridation entre ladite sonde et/ou lesdites amorces et l'acide nucléique de l'échantillon biologique à tester ;
c) les réactifs nécessaires à la mise en oeuvre d'une réaction d'amplification ;
d) les réactifs nécessaires à la détection et/ou au dosage de l'hybride formé entre ladite sonde et l'acide nucléique de l'échantillon biologique ou des acides nucléiques amplifiés formés.

Une telle trousse peut également contenir des contrôles positifs ou négatifs afin d'assurer la qualité des résultats obtenus. Elles peuvent également contenir les réactifs nécessaires à la préparation des acides nucléiques à partir de l'échantillon biologique.

Un autre objet de l'Invention est une puce à ADN comprenant au moins un polynucléotide selon l'Invention.

Encore un autre objet de l'Invention est l'utilisation d'un polynucléotide tel que défini ci-dessus pour la préparation d'une puce à ADN. L'Homme du Métier sait en fonction du support sélectionné, choisir la technique de réalisation appropriée pour réaliser une telle puce, comme par exemple par dépôt d'oligonucléotides sur support de verre ou de nylon, soit par greffage chimique ou électrochimique d'oligonucléotides.

Le polynucléotide de l'Invention peut être utilisé *in vitro*, comme moyen d'étude :
a) de la surexpression du transporteur (ZnT-8) dans des lignées de cellules modèles (insulinome de rat INS-1 par exemple) et de l'impact sur la sécrétion de l'insuline en réponse à une stimulation par le glucose ;
b) de la sensibilité des cellules bêta des îlots pancréatiques de Langerhans à la mort cellulaire (apoptose) induite par des conditions de stress oxydant ou de concentration faible ou forte en zinc ;
c) des étapes de différenciation de cellules souches en cellules sécrétrices d'insuline en réponse à différentes stimulations exogène (facteurs de croissance, extraits pancréatiques).

L'Invention concerne également la protéine codée par le polynucléotide selon l'Invention.

Par "protéine" on entend, au sens de la présente Invention, désigner un enchaînement précis d'acides aminés, modifiés ou non, comportant ou non des acides aminés non naturels.

La protéine selon l'Invention est obtenue soit à partir d'une cellule bêta, soit par synthèse chimique, soit par les techniques d'ADN recombinant, notamment à partir de vecteur d'expression comprenant un insert constitué par polynucléotide tel que défini ci-dessus.

L'Invention concerne aussi l'utilisation d'un polynucléotide tel que défini ci-dessus, pour la production d'une protéine ZnT-8 telle que définie ci-dessus.

La protéine selon la présente Invention lorsqu'elle est obtenue par synthèse chimique, peut être obtenue par l'une des nombreuses voies de synthèses peptidiques connues, par exemple les techniques mettant en oeuvre des phases solides ou des techniques utilisant des phases solides partielles, par condensation de fragments ou par une synthèse en solution classique. Dans ce cas la séquence de la protéine peut être modifiée afin d'améliorer sa solubilité, en particulier dans les solvants aqueux. De telles modifications sont connues de l'Homme du Métier comme par exemple la délétion de domaines hydrophobes ou la substitution d'acides aminés hydrophobes par des acides aminés hydrophiles.

De préférence, une protéine selon l'Invention est une protéine comprenant ou présentant la séquence SEQ ID NO:2 (correspondant à la protéine codée par le gène *ZnT*-*8*).

L'invention a également pour objet un fragment de la protéine telle que définie ci-dessus, caractérisé en ce qu'il est sélectionné dans le groupe constitué par les séquences SEQ ID NO:7, SEQ ID NO:8, SEQ ID NO:9 et SEQ ID NO:10.

Un autre objet de l'Invention est une puce à protéine comprenant une protéine ou un fragment de protéine tels que définis ci-dessus.

Encore un autre objet de l'Invention est l'utilisation d'une protéine ou d'un fragment de protéine tels que définis ci-dessus, pour la préparation d'une puce à protéine. Comme pour les puces a ADN, l'Homme du Métier sait en fonction du support choisi, choisir la technique de réalisation appropriée pour réaliser une telle puce.

La protéine, le fragment de protéine ou la puce à protéine tels que définis ci-dessus peuvent être utilisées pour détecter la présence d'anticorps dirigés contre lesdites protéines, dans le sérum d'un individu.

L'Invention concerne aussi l'utilisation de la protéine ou du fragment de protéine tel que définis ci-dessus, pour des mesures par des méthodes immuno-chimiques et immunoenzymatiques, ainsi que la recherche d'auto-anticorps dirigés contre la protéine selon l'Invention.

L'Invention a également pour objet un vecteur de clonage et/ou d'expression dans lequel est inséré le polynucléotide de l'Invention.

Un tel vecteur peut contenir les éléments nécessaires à l'expression et éventuellement à la sécrétion de la protéine dans une cellule hôte.

Lesdits vecteurs comportent de préférence : un promoteur, des signaux d'initiation et de terminaison de la traduction, ainsi que des régions appropriées de régulation de la transcription. Ils doivent pouvoir être maintenus de façon stable dans la cellule et peuvent éventuellement comprendre des séquences codant pour des signaux particuliers spécifiant la sécrétion de la protéine traduite tels que par exemple un promoteur fort de nature ubiquitaire, ou un promoteur sélectif d'un type de cellule et/ou de tissu particulier, comme par exemple le pancréas. Ces différentes séquences de contrôle sont choisies en fonction de l'hôte cellulaire utilisé.

Le polynucléotide selon l'Invention peut être inséré dans des vecteurs à réplication autonome au sein de l'hôte choisi ou des vecteurs intégratifs de l'hôte choisi.

Parmi les systèmes à réplication autonome, on utilise de préférence en fonction de la cellule hôte, des systèmes de type plasmidique ou viral. Les vecteurs viraux peuvent notamment être des adénovirus, des rétrovirus, des lentivirus, des poxvirus ou des virus herpétiques. L'Homme du Métier connaît les technologies utilisables pour chacun de ces systèmes.

Lorsque l'on souhaite l'intégration de la séquence dans les chromosomes de la cellule hôte, on peut utiliser par exemple des systèmes de type plasmidique ou viral; de tels virus sont, par exemple, les rétrovirus, ou les virus associés aux adénovirus (Adeno-associated virus ou AAV).

Parmi les vecteurs non viraux, on préfère les polynucléotides nus tels que l'ADN ou l'ARN nu, les chromosomes artificiels de bactérie (BAC, bacterial artificiel chromosome), les chromosomes artificiels de levure (YAC, yeast artificiel chromosome) pour l'expression dans la levure, les chromosomes artificiels de souris (MAC, mouse artificial chromosome) pour l'expression dans les cellules murines et de manière préférée les chromosomes artificiels d'homme (HAC, human artificial chromosome) pour l'expression dans les cellules humaines.

De tels vecteurs sont préparés selon les méthodes couramment utilisées par l'Homme du Métier, et les vecteurs recombinants en résultant peuvent être introduits dans l'hôte approprié par des méthodes standards, telles que par exemple la lipofection, l'électroporation, le choc thermique, la transformation après perméabilisation chimique de la membrane, la fusion cellulaire.

L'Invention a aussi pour objet les cellules hôtes modifiées, notamment les cellules eucaryotes et procaryotes, dans lesquelles au moins un polynucléotide selon l'Invention ou au moins un vecteur selon l'Invention a été introduit.

Parmi les cellules utilisables aux sens de la présente Invention, on peut citer les cellules bactériennes, les cellules de levure, les cellules animales, en particulier les cellules de mammifères ou encore les cellules végétales. On peut citer également les cellules d'insectes dans lesquelles on peut utiliser des procédés mettant par exemple en oeuvre des baculovirus.

L'Invention a également pour objet les organismes non-humains transgéniques tels que les animaux ou les végétaux transgéniques, dont tout ou partie des cellules contient le polynucléotide selon l'Invention ou le vecteur de l'Invention, sous une forme libre ou intégrée.

De préférence selon l'Invention, les organismes non-humains transgéniques sont ceux porteurs de cellules contenant un polynucléotide selon l'Invention non fonctionnel ou porteur d'une mutation.

Selon l'Invention les animaux transgéniques sont de préférence des mammifères, plus préférentiellement les rongeurs, en particulier les souris, les rats ou les lapins et les suidés, en particulier le porc.

Les animaux transgéniques peuvent être obtenus par toute méthode classique connue de l'Homme du Métier, comme par exemple par recombinaison homologue dans cellules souches embryonnaires, transfert de ces cellules souches à des embryons, sélection des chimères affectées au niveau des lignées reproductrices, et croissance desdites chimères.

Les cellules, animaux ou végétaux transgéniques selon l'Invention peuvent ainsi exprimer ou surexprimer le gène codant pour la protéine selon l'Invention ou leur gène homologue ou exprimer ledit gène dans lequel est introduite une mutation.

Les animaux transgéniques peuvent être utilisés par exemple en temps que modèles pour l'étude de l'étiologie des diabètes.

Les organismes transgéniques selon l'Invention peuvent être utilisés pour la production de la protéine selon l'Invention.

La protéine selon l'Invention peut être purifiée selon les techniques connues de l'Homme du Métier. Ainsi, la protéine peut être purifiée à partir de lysats et extraits cellulaires, du surnageant du milieu de culture, par des méthodes utilisées individuellement ou en combinaison, telles que le fractionnement, les méthodes de chromatographie, les techniques d'immunoaffinité à l'aide d'anticorps monoclonaux ou polyclonaux spécifiques, etc.. De préférence la protéine selon l'Invention est purifiée selon une méthode comprenant une première étape de séparation des protéines membranaires par centrifugation suivie d'une deuxième étape de purification par immunoaffinité selon la méthode décrite par T.C. Thomas, M.G. McNamee, (Purification of membrane proteins. Section IX, pp 499-520, in Methods in Enzymology, Guide to Protein Purification, Edité par M.P. Deutscher, vol 182, Academic press, New-York, 1990").

L'Invention a également pour objet une méthode de préparation de la protéine ZnT-8 recombinante, caractérisée en ce qu'elle comprend la culture des modifiées de la présente Invention, notamment les cellules de mammifères ou des cellules issues des organismes non-humains transgéniques selon l'Invention, dans des conditions permettant l'expression de ladite protéine, et la purification de ladite protéine recombinante.

L'Invention a également pour objet une protéine, caractérisée en ce qu'elle est susceptible d'être obtenue par l'une quelconque des méthodes de préparation ci-dessus décrites.

La protéine obtenue comme indiqué ci-dessus, peut aussi bien se présenter sous forme glycosylée que non glycosylée et peut présenter ou non la structure tertiaire de la protéine naturelle.

Les Inventeurs ont également pu montrer, par une étude du potentiel transmembranaire, que la structure secondaire de la protéine de l'Invention présente 3 boucles d'acides aminés extracellulaires (acides aminés 96-106, 164-176, 241-245) contre lesquelles peuvent être produits des anticorps monoclonaux ou polyclonaux.

L'Invention a ainsi également pour objet des anticorps mono- ou polyclonaux, caractérisés en ce qu'ils sont capables de reconnaître spécifiquement une protéine selon l'Invention.

De préférence, les anticorps reconnaissent spécifiquement la protéine de séquence SEQ ID NO:2, ses fragments ou les variants de ladite protéine tels que définis ci-dessus.

De manière préférée, les anticorps selon l'Invention reconnaissent spécifiquement les boucles extracellulaires de la protéine selon l'Invention correspondant aux SEQ ID NO:7, SEQ ID NO:8 et SEQ ID NO:10 (PEP1, PEP2 et PEP4) et/ou une boucle intracellulaire de la protéine selon l'Invention correspondant à la SEQ ID NO:9 (PEP3).

Les anticorps selon l'Invention sont, par exemple, des anticorps chimériques, des anticorps humanisés, des fragments Fab ou F(ab')2. Ils peuvent également se présenter sous forme d'immunoconjugués ou d'anticorps marqués afin d'obtenir un signal détectable et/ou quantifiable.

Lesdits anticorps peuvent être obtenus directement à partir de sérum humain, ou à partir de sérum d'animaux immunisés avec les protéines selon l'Invention, notamment celles produites par recombinaison génétique ou par synthèse peptidique.

Les anticorps polyclonaux spécifiques peuvent être obtenus selon les modes opératoires usuels. Les anticorps monoclonaux spécifiques peuvent être obtenus selon la méthode classique de culture d'hybridomes.

Un autre objet de l'Invention est une puce à protéine comprenant au moins un anticorps selon l'Invention.

L'Invention concerne aussi l'utilisation d'un anticorps selon l'Invention pour la préparation d'une puce à protéine comprenant ledit anticorps. L'Homme du Métier sait en fonction du support choisi, choisir la technique de réalisation appropriée pour réaliser une telle puce.

L'Invention a également pour objet l'utilisation des anticorps ou d'une puce à anticorps selon l'Invention pour la détection et/ou la purification d'une protéine selon l'Invention, de préférence des boucles extracellulaires ou intracellulaires de ladite protéine, de manière préférée des séquences correspondant aux SEQ ID NO:7 à SEQ ID NO:10.

De manière générale, les anticorps de l'Invention peuvent être avantageusement utilisés dans toute situation où l'expression d'une protéine selon l'Invention, normale ou mutée, doit être observée.

Particulièrement, les anticorps monoclonaux, peuvent être utilisés pour la détection de ces protéines dans un échantillon biologique. Ils constituent ainsi un moyen d'analyse immunocytochimique ou immunohistochimique de l'expression des protéines selon l'Invention, notamment la protéine de séquence SEQ ID NO:2 ou l'une de ses variantes, sur des coupes de tissus spécifiques. Généralement pour de telles analyses les anticorps utilisés sont marqués afin d'être détectables par exemple par des composés immunofluorescents, par marquage à l'or ou sous forme d'immunoconjugués enzymatiques.

Ils peuvent permettre notamment de mettre en évidence une expression anormale de ces protéines dans les tissus ou prélèvements biologiques.

Les Inventeurs décrivent aussi une méthode de détection dans un échantillon biologique de la protéine ZnT-8, comprenant une première étape de mise en contact de l'échantillon biologique avec un anticorps selon l'Invention et une deuxième étape de mise en évidence par tout moyen approprié du complexe antigène-anticorps formé.

L'Invention a également pour objet une trousse permettant de mettre en oeuvre le procédé ci-dessus décrit comprenant :
a) au moins un anticorps monoclonal ou polyclonal selon l'Invention ;
b) les réactifs permettant la détection du complexe antigène-anticorps produit lors de la réaction immunologique.

Selon un mode de réalisation particulier, la trousse peut éventuellement comprendre des réactifs pour la constitution d'un milieu permettant la réaction immunologique.

Les anticorps selon l'Invention peuvent également être utilisés pour la détection et/ou le tri des îlots de Langerhans, préférentiellement des cellules bêta, à partir de pancréas humain ou animal, notamment le pancréas de souris, rat, lapin et de porc. Ce tri peut être réalisé par un appareillage de cytométrie en flux (FACS) pour ce qui concerne les cellules isolées. Pour les îlots, leur marquage pourrait améliorer les méthodes de séparation actuelle : séparation par gradient de densité avec du Ficoll, de l'Euro-Ficoll ou du Ficoll-Diatrizoate de sodium ou la méthode de choix qui est un gradient d'albumine sur un séparateur de cellules.

Ainsi, l'Invention a pour objet une méthode de sélection des cellules bêta des îlots de Langerhans, comprenant une première étape de mise en contact des cellules d'un échantillon biologique susceptible de contenir de tels îlots et/ou cellules avec une anticorps selon l'Invention, une deuxième étape de mise en évidence par tout moyen approprié des cellules marquées par l'anticorps et une troisième étape d'isolement par tout moyen approprié des cellules marquées.

Les anticorps selon l'Invention peuvent également être utilisés pour suivre le processus de différenciation de cellules souches en cellules bêta des îlots de Langerhans, particulièrement humaines ou animales, ainsi que de trier ces cellules exprimant la protéine ZnT-8, particulièrement la protéine de séquence SEQ ID NO:2 après différenciation.

Ainsi, il est aussi décrit une méthode pour suivre le processus de différenciation de cellules souches en cellules d'îlot pancréatique ou en cellules bêta, comprenant une étape de mise en contact des cellules d'un échantillon biologique susceptible de contenir lesdites cellules souches en cours de différenciation avec un anticorps selon l'Invention, une deuxième étape de mise en évidence par tout moyen approprié des cellules marquées par l'anticorps et une troisième étape de visualisation par tout moyen approprié des cellules marquées.

Ladite méthode peut en outre comprendre une étape supplémentaire d'isolement par tout moyen approprié des cellules marquées.

Le polynucléotide, la cellule, l'organisme transgénique ou la puce à ADN selon l'Invention peuvent être utilisés pour le criblage de composés chimiques ou biochimiques pouvant interagir *in vitro ou in vivo*, directement ou indirectement, avec le polynucléotide selon l'Invention et/ou moduler l'expression dudit polynucléotide.

Ainsi, l'Invention a pour objet une méthode de criblage d'un composé chimique ou biochimique pouvant interagir *in vitro ou in vivo*, directement ou indirectement avec le polynucléotide selon l'Invention caractérisée en ce qu'elle comprend une première étape de mise en contact d'un composé chimique ou biochimique candidat avec le polynucléotide, la cellule, l'organisme non-humain transgénique ou la puce à ADN selon l'Invention, et une deuxième étape de détection du complexe formé entre le composé chimique ou biochimique candidat et le polynucléotide, la cellule, l'organisme non-humain transgénique ou la puce à ADN selon l'Invention.

L'Invention a aussi pour objet une méthode de criblage d'un composé chimique ou biochimique pouvant moduler *in vitro ou in vivo,* directement ou indirectement, l'expression du polynucléotide selon l'Invention, caractérisée en ce qu'elle comprend une première étape de mise en contact d'un composé chimique ou biochimique candidat avec le polynucléotide, la cellule, l'organisme non-humain transgénique ou la puce à ADN selon l'Invention, et une deuxième étape de mesure par tout moyen approprié de l'expression dudit polynucléotide.

La protéine, la cellule, l'organisme transgénique ou la puce à protéine selon l'Invention peuvent être utilisés pour le criblage de composés chimiques ou biochimiques pouvant interagir *in vitro ou in vivo*, directement ou indirectement, avec la protéine selon l'Invention, et/ou qui peuvent moduler l'expression ou l'activité de ladite protéine.

Ainsi, l'Invention a pour objet une méthode de criblage d'un composé chimique ou biochimique pouvant interagir *in vitro ou in vivo* directement ou indirectement, avec la protéine selon l'Invention, caractérisée en ce qu'elle comprend une première étape de mise en en contact d'un composé chimique ou biochimique candidat avec la protéine, la cellule, l'organisme non-humain transgénique ou la puce à protéine selon l'Invention, une deuxième étape, de détection du complexe formé entre le composé chimique ou biochimique candidat et la protéine, la cellule, l'organisme non-humain transgénique ou la puce à protéine selon l'Invention.

L'Invention a aussi pour objet une méthode de criblage d'un composé chimique ou biochimique pouvant moduler *in vitro ou in vivo* directement ou indirectement, l'expression et/ou l'activité de la protéine selon l'Invention, caractérisée en ce qu'elle comprend une première étape de mise en contact d'un composé chimique ou biochimique candidat avec la protéine, la cellule, l'organisme non-humain transgénique ou la puce à protéine selon l'Invention, et une deuxième étape de mesure par tout moyen approprié de l'expression et/ou de l'activité de ladite protéine.

L'Invention a également pour objet le polynucléotide, la protéine, les anticorps, les vecteurs ou les cellules transformées, selon l'Invention, comme médicaments.

Le polynucléotide, la protéine, les anticorps, les vecteurs ou les cellules transformées, selon l'Invention, peuvent être utilisés dans la préparation d'un médicament destiné à la prévention et/ou au traitement du diabète, particulièrement celui associé à la présence d'au moins une mutation du gène correspondant à SEQ ID NO:1, et/ou à une expression anormale de la protéine correspondant à SEQ ID NO:2, ou destiné à la prévention et/ou au traitement des hyperinsulinismes lorsque l'on observe une expression, une maturation ou une sécrétion anormale du gène de l'insuline, ou destiné à réguler la maturation et la sécrétion de l'insuline dans les cellules bêta et ou dans des cellules modifiées en vue d'une sécrétion d'insuline ou destiné à réguler les phénomènes d'apoptose des cellules bêta.

Une expression anormale signifie une surexpression, une sous-expression ou l'expression d'une protéine mutée. Une maturation anormale signifie une protéolyse absente ou insuffisante de la pro-insuline en insuline ou une co-cristallisation absente, insuffisante ou trop importante de l'insuline et du zinc dans les vésicules de sécrétion intracellulaires.

L'Invention a également pour objet l'utilisation d'un polynucléotide, d'une protéine ou d'un anticorps selon l'Invention pour déterminer une variabilité allélique, une mutation, une délétion, une perte d'hétérozygotie ou toute anomalie génétique du gène codant pour la protéine selon l'Invention. On peut détecter les mutations dans la séquence du gène *ZnT*-*8* directement par analyse de l'acide nucléique et des séquences selon l'Invention (ADN génomique, ARN, ou ADNc), mais également par l'intermédiaire de la protéine selon l'Invention. En particulier, l'utilisation d'un anticorps selon l'Invention qui reconnaît un épitope portant une mutation permet de différencier une protéine "saine" d'une protéine "associée à une pathologie".

L'Homme du Métier sait également mettre en oeuvre des techniques permettant l'étude de l'altération de l'expression d'un gène, par exemple par analyse de l'ARNm, en particulier par Northem Blot ou par RT-PCR avec des sondes ou des amorces selon l'Invention, ou bien par analyse de la protéine exprimée, en particulier par Western Blot, en utilisant des anticorps selon l'Invention.

Outre les dispositions qui précèdent, l'Invention comprend encore d'autres dispositions qui ressortiront de la description qui va suivre, qui se réfère à des exemples de mise en oeuvre de l'Invention ainsi qu'aux dessins annexés, dans lesquels :

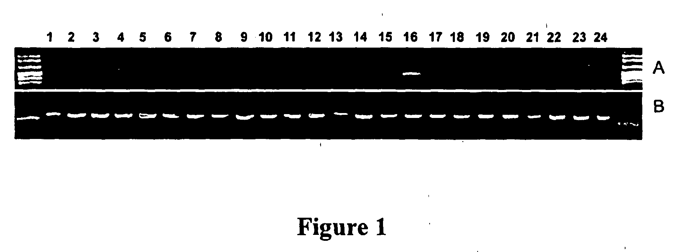

- la figure 1 illustre l'analyse par RT-PCR de l'expression tissulaire de l'ARN messager codant pour la protéine ZnT-8 (A), par comparaison avec l'expression du messager ubiquitaire de l'actine (B). 1 : Cerveau, 2 : Coeur, 3 : Rein, 4 : Rate, 5 : Foie, 6 : Colon, 7 : Poumon, 8 : intestin grêle, 9 : Muscle, 10 : Estomac, 11 : Testicule, 12 : Placenta, 13 : Glandes salivaires, 14 : Thyroïde, 15 :Glandes surrénales, 16 : Pancréas, 17 : Ovaires, 18 : Utérus, 19 : Prostate, 20 : Peau, 21 : Leucocytes, 22 : Moelle osseuse, 23 : Cerveau foetal, 24 : Foie foetal. L'expression dudit ARNm est détecté uniquement dans le pancréas (piste 16).

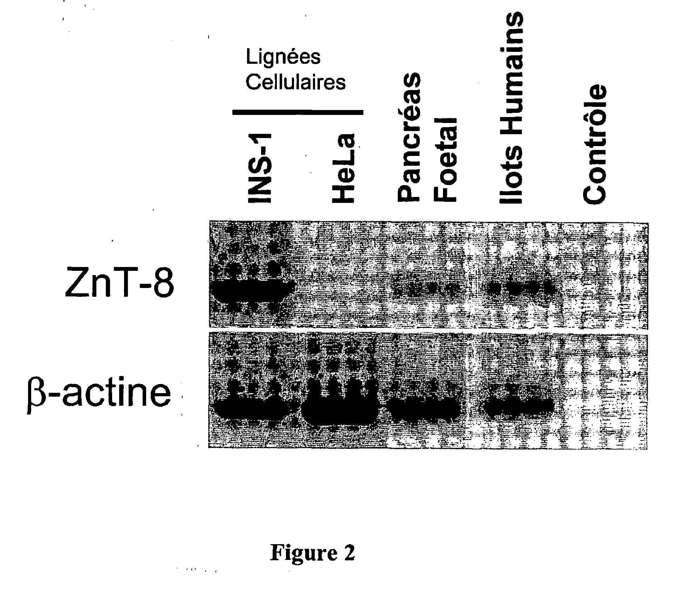

- la figure 2 illustre l'analyse par RT-PCR de l'expression de l'ARN messager codant pour la protéine ZnT-8, dans : une lignée d'insulinome de rat (INS-1, piste 1), des îlots pancréatiques humains foetaux (piste 2) et adultes (piste 3), par comparaison avec une lignée de cellules épithéliales (lignée Hela). L'ARNm est détecté dans la lignée d'insulinome de rat et les îlots pancréatiques adultes et foetaux, alors qu'aucun transcrit n'est détecté dans les cellules épithéliales (lignée Hela). Le messager de l'actine est utilisé comme contrôle.

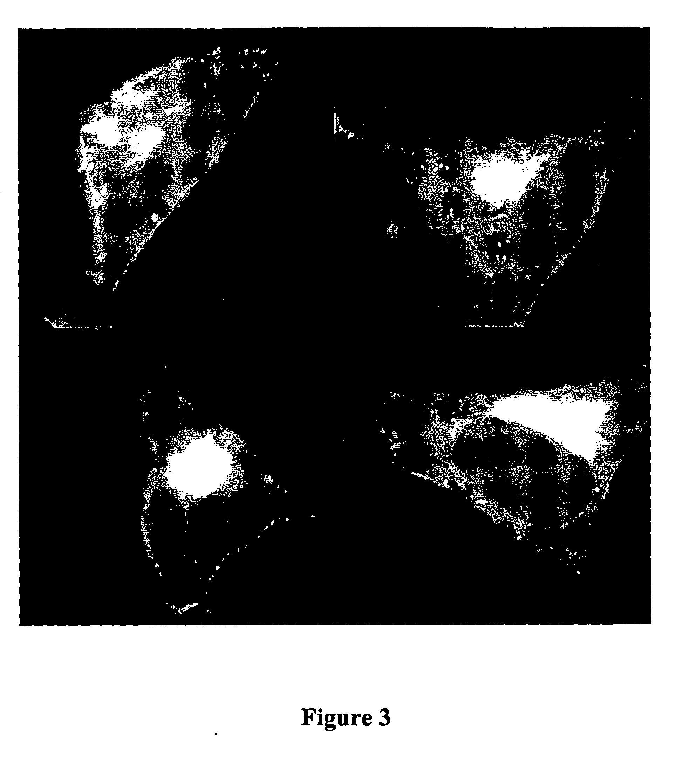

- la figure 3 illustre l'analyse en microscopie à fluorescence, de la localisation de la protéine de fusion ZnT-8-GFP dans des cellules épithéliales (lignée hela) transfectées. La fluorescence est localisée dans les vésicules intracytoplamiques, ainsi qu'au niveau de la membrane plasmique.

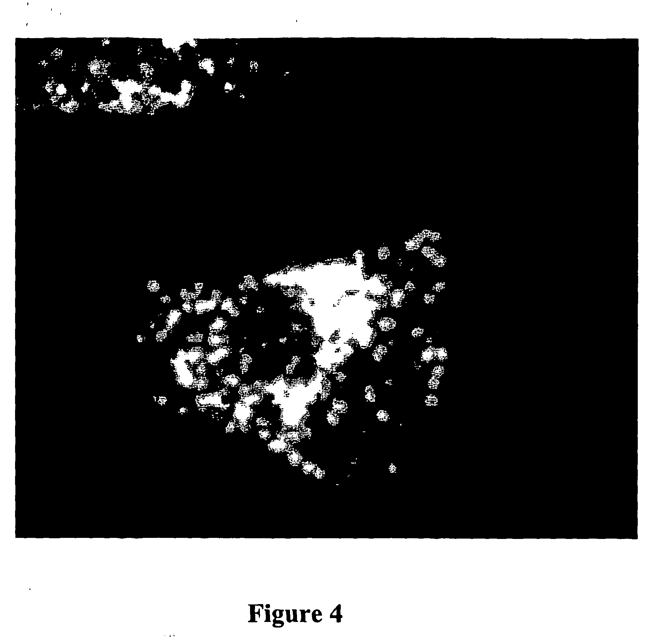

- la figure 4 illustre l'analyse en microscopie à fluorescence, de la localisation de la protéine de fusion ZnT-8-GFP dans des cellules d'insulinome de rat (lignée INS-1) transfectées. La fluorescence est localisée dans les vésicules de sécrétion indiquant un rôle de Znt-8 dans la maturation et l'exocytose de l'insuline.

Les exemples suivants sont illustratifs de l'Invention et ne la limitent aucunement.

### EXEMPLE 1 : Clonage de l'ADNc codant pour la protéine Znt-8

Le gène codant pour la protéine Znt-8, dénommé gène *Znt-8*, a été identifié par bioinformatique, par recherche de gènes homologues à ceux de la famille *ZnT*, en utilisant les séquences du génome humain disponibles. L'analyse des séquences génomiques a permis de localiser et de définir l'organisation intron/exon du gène *Znt-8*.

L'ADNc codant pour ZnT-8 a été amplifiée par RT-PCR à partir d'ARNm d'îlots de pancréas humains préparés selon la technique donnée dans Kenmochi T. et al. (Pancreas, 2000, 20, 2, 184-90), à l'aide de la paire d'amorces (SEQ ID NO:5 : 5'-ACTCTAGAATGGAGTTTCTTGAAAGAACGT A et SEQ ID NO:6 : 5'-AATCTA GAGTCACAGGGGTCTTCACAGA.), définies à partir de la séquence du gène *Znt-8*.

De manière plus précise, les ARN totaux ont été extraits à partir des îlots, en utilisant le kit "RNA extraction kit" (Roche) selon les instructions du fabricant. Les ARN ainsi obtenus ont été dosés par mesure de l'absorbance à 250 nm et conservés à -80°C.

L'amplification a été réalisée en utilisant le kit "Titan one tube RT-PCR kit" (Roche) selon les instruction du fabriquant, et la paire d'amorces SEQ ID NO:5 et SEQ ID NO:6. La transcription inverse a été réalisée à 52°C pendant 30 min puis les ADNc synthétisés ont été amplifies par 30 cycles (30 s à 94°C, 30 s à 53°C, 1 min à 72°C) et une élongation finale de 5 min à 72°C. Les produits d'amplification ont été séparés par électrophorèse en gel d'agarose (1,5 %) en présence de bromure d'éthydium et le produit d'amplification de 1123 paires de bases comprenant la séquence d'ADNc présentant la séquence SEQ ID NO:1 a été purifié à l'aide du kit Nucleic acid purification kit (QIAGEN), en suivant les instructions du fabricant.

### EXEMPLE 2 : Analyse de l'expression tissulaire de l'ARN messager codant pour la protéine Znt-8

L'expression de l'ARN messager codant pour la protéine Znt-8 a été analysée par PCR sur des ADNc commerciaux préparés à partir de différents tissus humains en utilisant les amorces suivantes : SEQ ID NO:3 : 5'-GAT GCT GCC CAC CTC TTA ATT GAC et SEQ ID NO:27 : CCA AGA CCA GGA TGG TTT AGA TGA (séquence complémentaire de la SEQ ID NO:4 : 5'-TCA TCT TTT CCA TCC TGG TCT TGG). Les amorces (SEQ ID NO:3 et SEQ ID NO:27) ont été choisies dans 2 exons différents pour éviter l'amplification d'une séquence génomique. Les tissus testés sont : 1 : Cerveau, 2 : Coeur, 3 : Rein, 4 : Rate, 5 : Foie, 6 : Colon, 7 : Poumon, 8 : intestin grêle, 9 : Muscle, 10 : Estomac, 11 : Testicule, 12 : Placenta, 13 : Glande salivaire, 14 : Thyroïde, 15 : Corticosurrénale, 16 : Pancréas, 17 : Ovaire, 18 : Utérus, 19 : Prostate, 20 : Peau, 21 : Leucocytes sanguins, 22 : Moelle osseuse, 23 : Cerveau foetal, 24 : Foie foetal.

De manière plus précise : 2 µl d'ADNc ont été mélangés avec les 2 amorces spécifiques (1µM final) et un mélange classique de PCR (1 unité de Taq DNA polymerase, tampon avec 1,5 mM magnésium, dNTP 10 mM). L'amplification a été réalisée par 30 cycles (30 s à 94°C, 30 s à 53°C, 1 min à 72°C) et une étape finale d'élongation de 5 min à 72°C. Les produits ont ensuite été analysés par électrophorèse en gel d'agarose (1,5 %) en présence de bromure d'éthydium.

Les résultats illustrés par la figure 1 montrent que par comparaison avec le messager de l'actine utilisé comme contrôle (figure 1B), l'ARNm correspondant au polynucléotide de l'Invention est exprimé uniquement dans les cellules de pancréas (piste 16 de la figure 1A) mais pas dans les cellules des 23 autres tissus analysés (Pistes 1 à 15 et 17 à 24 de la figure 1A).

### EXEMPLE 3 : Expression de l'ARN messager correspondant au polynucléotide de l'Invention dans des cellules de pancréas foetal et adulte et dans une lignée d'insulinome de rat (INS-1)

L'analyse de l'expression de l'ARN messager codant pour la protéine ZnT-8 a été réalisée par RT-PCR, à partir d'ARN de différents tissus humains : îlots pancréatiques humains adultes et foetaux, lignée d'insulinome de rat (INS-1 ; Asfari M. et al., Endocrinology, 1992, 130, 1, 167-78), par comparaison avec une lignée de cellules épithéliales humaines (Hela), utilisée comme contrôle. 10⁶ cellules sont lavées deux fois avec du tampon phosphate (PBS) puis centrifugées 3 min à 2000 g. Les ARN totaux sont extraits comme décrit à l'exemple 1 et la concentration en ARN est ajustée à 1 ng/µl pour ZnT-8 ou 1 pg/µl pour le contrôle β-actine. Les transcrits sont amplifiés puis les produits d'amplification sont analysées, comme décrit à l'exemple 2.

Les résultats illustrés par la figure 2, montrent que l'ARNm codant pour la protéine ZnT-8 est exprimé dans les cellules des îlots pancréatiques humains foetaux et adultes et dans une lignée d'insulinome de rat (INS-1) mais pas dans une lignée de cellules épithéliales (lignée Hela).

### EXEMPLE 4 : Expression d'une protéine de fusion ZnT-8/GFP

La protéine est la protéine humaine, codée par l'ADNc correspondant à la séquence SEQ ID NO:1 (ZnT-8). Le produit PCR de 1123 paires de bases obtenu selon l'exemple 1 a été digéré avec l'enzyme de restriction *XbaI*, puis cloné dans le vecteur pcDNA3.1-CT-GFP (Invitrogen), pour donner le vecteur dénommé pZnT-8-GFP qui a été vérifié par séquençage.

Le vecteur pZnT-8-GFP a été transfecté de façon transitoire dans une lignée de cellules épithéliales (Hela) et de façon stable dans une lignée d'insulinome de rat (INS-1).

Les cellules épithéliales HeLa (ATCC numéro CCL-2) sont cultivées dans le milieu Opti-MEM (Modified Eagle's Medium, Life Technologies) supplémenté avec 5 % de sérum de veau décomplémenté et 2 mM de glutamine. Les cellules sont incubées à 37°C dans une atmosphère humidifiée enrichie à 5 % en CO₂.

Les cellules INS-1 sont cultivées dans du milieu RPMI (Life Technologies) supplémenté avec : sérum de veau foetal (10 %), 2-Mercaptoéthan-1-ol (50 µM), pyruvate de sodium (1 mM), HEPES (10 mM), L-glutamine (2 mM), penicilline 100 U/mL et streptomycine (100 µg/ml).

Les cellules cultivées dans des boites de Pétri de 35 mm sont transfectées avec le vecteur pZnT-8-GFP (1 µg d'ADN pour 10⁶ cellules), en utilisant le réactif Exgen500 (Euromedex) selon les instructions du fabricant. Après transfection par le vecteur ZnT-8-GFP, les cellules INS-1 sont sélectionnées, clonées dans le même milieu que précédemment additionné de 400 µg/mL de G418, puis la fluorescence des clones est observée sous un microscope à fluorescence inversé (Axiovert, Zeiss) en utilisant les paramètres suivants : longueur d'onde d'excitation : 450-490 nm ; longueur d'onde d'émission : 520 nm.

L'expression de la protéine de fusion ZnT-8-GFP dans les cellules Hela est analysée 48 heures après la transfection, par observation de la fluorescence comme précisé ci-dessus pour les clones de cellules INS-1 transfectées de façon stable.

Les résultats illustrés par la figure 3 (lignée Hela) montrent que la protéine ZnT-8 est localisée dans des vésicules intracytoplamiques, ainsi que sur la membrane plasmique ; cette localisation démontre que la protéine ZnT-8 emprunte la voie d'excrétion extracellulaire et se retrouve à la surface de la cellule.

Les résultats illustrés par la figure 4 (lignée INS-1) montrent que la protéine ZnT-8 est localisée dans les vésicules de sécrétion de l'insuline ; cette localisation indique que ZnT-8 est impliquée dans la maturation de l'insuline ; en outre, dans la mesure où cette expérience est réalisée à l'état basal (en l'absence de stimulation par le glucose) la protéine sera également présente sur la membrane plasmique au cours de l'exocytose de l'insuline, après stimulation par le glucose.

### EXEMPLE 5 : Analyse de la séquence de la protéine ZnT-8

L'analyse de la séquence primaire de ZnT-8 et la prédiction des domaines transmembranaires ont été réalisées avec les programmes TMpred (*http* ://*www.ch.embnet.org*/*softwarelTMPRED_form.html*) et SOSUI (*http* ://*sosui.proteome.bio.tuat.ac.jp*/*sosuimenu0.html*).

La protéine ZnT-8 complète possède 6 domaines transmembranaires prédits (acides aminés 74-95, 107-126, 141-163, 177-196, 216-240, 246-267), les extrémités N- et C-terminales étant situées dans le cytoplasme.

### EXEMPLE 6 : Préparation d'un anticorps polyclonal dirigé contre les boucles extracellulaires (PEP1, PEP2 et PEP4) et contre une boucle intracellulaire (PEP3) de ZnT-8

Les peptides correspondant aux épitopes présentant les séquences SEQ ID NO:7 : PEP1 : HIAGSLAVVTDAAHLL ; SEQ ID NO:8 : PEP2 : CERLLYPDYQIQATV ; SEQ ID NO:9 : PEP3 CLGHNHKEVQANASVR, SEQ ID NO:10 : PEP4 : YFKPEYKIADPIC ont été synthétisés en phase solide, selon la méthode originellement décrite par Merrifield et al. (J. Am. Chem. Soc., 1964, 85: 2149-) (1964), purifiés et conjugués à une protéine porteuse (albumine, par exemple). Les peptides conjugués sont injecté à des lapins selon le protocole d'immunisation suivant :
J0 : première immunisation ; J14 : seconde immunisation ; J28 : Troisième immunisation ; J38 : vérification de la spécificité ; J56 : quatrième immunisation ; J66 et J80 : récupération du sérum. Le sérum, peut être utilisé directement ou après purification sur colonne de protéine A avec une élution en milieu acide. Ces opérations ont été effectuées à façon par la société Eurogentec SA, Belgique.

### EXEMPLE 7 : Marquage fluorescent de l'anticorps obtenu à l'exemple 6

L'anticorps est purifié par chromatographie d'affinité sur une colonne de protéine G (Pharmacia). La colonne (1ml), équilibrée avec un tampon phosphate de sodium 10 mM, NaCl 0,15 M, pH 7,4 est chargée avec 5 ml de sérum, puis lavée avec 20 ml du même tampon pour éluer les protéines non fixées. L'anticorps est ensuite décroché avec une solution de glycine, HCl 0,1M, pH 2,5, puis neutralisé par 40µl de tampon Tris HCl 2M, pH 10,0.

2 mg d'anticorps sont dilués dans 1 ml de tampon phosphate pH 8.0. Une solution de NHS-FITC (SIGMA ; 1mg/ml dans DMSO) est préparée extemporanément. 75 µl de la solution NHS-FITC est mélangé avec la solution d'anticorps puis le mélange est incubé à température ambiante pendant 45 minutes. L'anticorps marqué est purifié sur colonne PD-10 (PHARMACIA) de la façon suivante : la colonne est lavée avec 30ml de PBS, chargée avec 1ml de la solution d'anticorps marqué à purifier, puis avec 5 ml de PBS et des fractions de 2 ml sont ensuite collectés ; la seconde fraction contenant l'anticorps marqué est conservée.

### RÉFÉRENCES BIBLIOGRAPHIQUES

1. Soria B. : In-vitro differentiation of pancreatic beta-cells. Differentiation 2001 ; 68 : 205-19.
2. Gores PF, Sutherland DE. Pancreatic islet transplantation : is purification necessary ? Am. J. Surg. 1993 ; 166 : 538-42.
3. Bloc A, Cens T, Cruz H, Dunant Y. Zinc-induced changes in ionic currents of clonal rat pancreatic-cells : activation of ATP-sensitive K+ channels. J. Physiol. 2000 ; 529 Pt 3 : 723-34.
4. Meyer K, Irminger JC, Moss LG, de Vargas LM, Oberholzer J, Bosco D, et al. : Sorting human beta-cells consequent to targeted expression of green fluorescent protein. Diabetes 1998 ; 47 : 1974-7.
5. Giordano C, Stassi G, Todaro M, Richiusa P, Giordano M, Mattina A, et al. : Autofluorescence-activated sorting of human single beta cells and endocrine non-beta cells after enzymatic islet dissociation. Transplant Proc ; 1994 ; 26 : 651-2.
6. Lukowiak B, Vandewalle B, Riachy R, Kerr-Conte J, Gmyr V, Belaich S, et al. : Identification and purification of functional human beta-cells by a new specific zinc fluorescent probe. J. Histochem. Cytochem. 2001 ; 49 : 519-28.
7. Shiroi A, Yoshikawa M., Yokota H., Fukui H., Ishizaka S., Tatsumi K, et al. : Identification of Insulin-Producing Cells Derived from Embryonic Stem Cells by Zinc-Chelating Dithizone. Stem Cells 2002 ; 20 : 284-292.
8. Jiao L, Gray DW, Gohde W, Flynn GJ, Morris PJ. In vitro staining of islets of Langerhans for fluorescence-activated cell sorting. Transplantation 1991 ; 52 : 450-2.
9. Kallan AA, Roep BO, Arden SD, Hutton JC, de Vries RR. : Beta-cell reactive T-cell clones from type I diabetes patients are not beta cell specific and recognize multiple antigens. J. Autoimmun. 1995 ; 8 : 887-99.
10. Zalewski PD, Millard SH, Forbes IJ, Kapaniris 0, Slavotinek A, Betts WH et al. : Video image analysis of labile zinc in viable pancreatic islet cells using a specific fluorescent probe for zinc. J. Histochem. Cytochem. 1994 ; 42 : 877-84.
11. Easom RA. Beta-granule transport and exocytosis. Semin. Cell. Dev. Biol. 2000 ; 11 : 253-66.
12. Palmiter RD, Findley SD. Cloning and functional characterization of a mammalian zinc transporter that confers resistance to zinc. Embo J. 1995 ; 14 : 639-49.
13. Sambrook J, Russell DW. (2000) Molecular Cloning. A Laboratory Manual, Cold Spring Harbor Laboratory Press.
14. Bloc A et al, J Physiol. 2000, Dec. 15 ; 529 Pt 3 : 723-34)
15. Tomita T. Pathology International 2002 ; 52 : 425-432.

## Revendications

1. Utilisation en tant que marqueur spécifique des cellules bêta des îlots pancréatiques de Langerhans d'au moins un polynucléotide isolé ou de la protéine correspondante choisis parmi :
- les polynucléotides comprenant ou présentant l'une des séquences suivantes : (a) la séquence SEQ ID NO:1, (b) un fragment de la séquence SEQ ID NO:1 d'au moins 15 nucléotides consécutifs, (c) une séquence présentant un pourcentage d'identité d'au moins 80 % après alignement optimal avec l'une des séquences définies en (a) ou en (b) et, (d) une séquence complémentaire, sens ou antisens de l'une des séquences définies en (a), (b) ou (c), et
- les protéines codées par les polynucléotides tels que définis en (a), (b), (c) ou (d) ci-dessus comprenant ou présentant l'une des séquences suivantes : (e) la séquence SEQ ID NO:2, (f) un fragment de la séquence SEQ ID NO:2 d'au moins 15 acides aminés consécutifs, (g) une séquence présentant un pourcentage d'identité d'au moins 60 % après alignement optimal avec l'une des séquences définies en (e) ou en (f), de préférence 80 % d'identité, ou de manière encore plus préférée 90 % d'identité.

2. Utilisation selon la revendication 1, **caractérisée en ce que** ledit polynucléotide isolé tel que défini en c), est un polynucléotide variant de la séquence SEQ ID NO:1 1 comportant une mutation qui conduit à une modification de la séquence en acides aminés de la protéine codée par la séquence SEQ ID NO:1.

3. Utilisation selon la revendication 1, **caractérisée en ce que** ledit polynucléotide isolé tel que défini en b) ou en d) est choisi parmi la paire d'amorces SEQ ID NO:3 et SEQ ID NO:27 et la paire d'amorces SEQ ID NO:5 et SEQ ID NO:6.

4. Utilisation selon la revendication 1, **caractérisée en ce que** ledit polynucléotide isolé est susceptible d'être obtenu par amplification à l'aide de la paire d'amorces telle que définie à la revendication 3.

5. Utilisation selon la revendication 1, **caractérisée en ce que** ledit polynucléotide tel que défini en (d) est un petit ARN interférent (siRNA) qui par interaction avec les ARNm correspondants audit polynucléotide, conduira à leur dégradation.

6. Utilisation selon la revendication 1, **caractérisée en ce que** ladite protéine telle que définie en (g) est un variant de la séquence SEQ ID NO:2 présentant une mutation associée au diabète ou à l'hyperinsulinisme.

7. Utilisation selon la revendication 1, **caractérisée en ce que** ledit fragment tel que défini en (f) présente une séquence choisie parmi les séquences SEQ ID NO:7, SEQ ID NO:8, SEQ ID NO:9 et SEQ ID NO:10.

8. Polynucléotide isolé, **caractérisé en ce qu'**il est utilisable selon la revendication 1, et **en ce qu'**il comprend ou présente une séquence choisie parmi :
(a) la séquence SEQ ID NO:1,
(b) un fragment de la séquence SEQ ID NO:1 d'au moins 20 nucléotides consécutifs,
(c) une séquence complémentaire, sens ou antisens de l'une des séquences définies en (a) ou (b),
à l'exception des fragments d'au moins 15 nucléotides consécutifs inclus dans les séquences présentant les numéros d'accès dans la base de données NCBI n° AX526723 (SEQ ID NO : 11) , n° AX526725 (SEQ ID NO : 12) et n° AX526727 (SEQ ID NO : 13), des EST présentant les numéros d'accès dans la base de données GenBank BM565129 (SEQ ID NO : 14), BM310003 (SEQ ID NO : 15), BM875526 (SEQ ID NO : 16), BG655918 (SEQ ID NO : 17), BQ417284 (SEQ ID NO : 18), BQ267316 (SEQ ID NO : 19), BU072134 (SEQ ID NO : 20), BQ267526 (SEQ ID NO : 21), BQ270198 (SEQ ID NO : 22), BU581447 (SEQ ID NO : 23), BU070173 (SEQ ID NO : 24), BQ631692 (SEQ ID NO : 25) et BU949895 (SEQ ID NO : 26) ainsi que des séquences présentant les numéros d'accès dans la base de données NCBI AX526723 (SEQ ID NO : 11), AX526725 (SEQ ID NO : 12) et AX526727 (SEQ ID NO : 13).

9. Sonde pour détecter, identifier ou doser des acides nucléiques correspondants aux polynucléotides tels que définis à la revendication 1, **caractérisée en ce qu'**elle est constituée d'un polynucléotide selon la revendication 8.

10. Paire d'amorces pour l'amplification d'acides nucléiques utilisables selon la revendication 1, **caractérisée en ce qu'**elle est choisie parmi les paires d'amorces telles que définies à la revendication 3.

11. Polynucléotide isolé utilisable selon la revendication 1, susceptible d'être obtenu par amplification à l'aide des amorces selon la revendication 10.

12. Polynucléotide selon la revendication 8, **caractérisé en ce qu'**il s'agit d'un petit ARN interférent correspondant au polynucléotide tel que défini à la revendication 1, qui par interaction avec les ARNm correspondants audit polynucléotide, conduira à leur dégradation.

13. Méthode de détermination du profil de transcription du gène correspondant au polynucléotide selon la revendication 8 ou la revendication 11 ou d'une altération dudit profil, dans un échantillon biologique, comprenant une première étape d'obtention par tout moyen approprié des ARN à partir de l'échantillon biologique, une deuxième étape de mise en contact desdits ARN avec une sonde marquée constituée d'un polynucléotide selon l'une quelconque des revendications 8, 9 ou 11 dans des conditions appropriées d'hybridation entre les ARN et la sonde et une troisième étape de révélation par tout moyen approprié des hybrides formés.

14. Méthode selon la revendication 13, dans laquelle la deuxième étape est une étape de rétrotranscription et/ou d'amplification des transcrits, réalisée à l'aide d'une paire d'amorces selon la revendication 10 et la troisième étape est une étape de révélation par tout moyen approprié des acides nucléiques amplifiés.

15. Méthode selon l'une quelconque des revendications 13 ou 14, **caractérisée en ce qu'**elle comporte en outre une étape d'évaluation du taux de transcription du gène par comparaison avec un témoin préalablement choisi.

16. Méthode de mise en évidence du gène correspondant au polynucléotide selon la revendication 8 ou la revendication 11 ou des variants alléliques dudit gène ou d'une altération fonctionnelle de ce gène, dans un échantillon biologique, comprenant une première étape d'obtention par tout moyen approprié de l'ADN à partir de l'échantillon biologique, une deuxième étape de mise en contact desdits ADN avec une sonde marquée constituée d'un polynucléotide selon l'une quelconque des revendications 8, 9 ou 11 dans des conditions appropriées d'hybridation entre les ADN et la sonde et une troisième étape de révélation par tout moyen approprié des hybrides formés.

17. Méthode selon la revendication 16, dans laquelle la deuxième étape est une étape d'amplification réalisée à l'aide d'une paire d'amorces selon la revendication 10 et la troisième étape est une étape de révélation par tout moyen approprié des acides nucléiques amplifiés formés.

18. Méthode selon l'une quelconque des revendications 16 ou 17, **caractérisée en ce qu'**elle comporte en outre une étape d'isolement et de séquençage des acides nucléiques mis en évidence.

19. Trousse de réactifs pour la mise en oeuvre des méthodes selon l'une quelconque des revendications 13 à 18 comprenant :
a) au moins une sonde selon la revendication 9 et/ou une paire d'amorces selon la revendication 10 ;
b) les réactifs nécessaires à la mise en oeuvre d'une réaction d'hybridation entre ladite sonde et/ou lesdites amorces et l'acide nucléique de l'échantillon biologique ;
c) les réactifs nécessaires à la mise en oeuvre d'une réaction d'amplification ;
d) les réactifs nécessaires à la détection et/ou au dosage de l'hybride formé entre ladite sonde et l'acide nucléique de l'échantillon biologique ou des acides nucléiques amplifiés formés.

20. Puce à ADN comprenant au moins un polynucléotide selon la revendication 8 ou la revendication 11.

21. Utilisation d'un polynucléotide selon la revendication 8 ou la revendication 11 pour la préparation d'une puce à ADN.

22. Utilisation *in vitro* du polynucléotide selon l'une quelconque des revendications 8, 11 ou 12, comme moyen d'étude :
a) de la surexpression du transporteur codé par le polynucléotide selon la revendication 8 ou la revendication 11 dans des lignées de cellules modèles et de l'impact sur la sécrétion de l'insuline en réponse à une stimulation par le glucose ;
b) de la sensibilité des cellules bêta des îlots pancréatiques de Langerhans à la mort cellulaire (apoptose) induite par des conditions de stress oxydant ou de concentration faible ou forte en zinc ;
c) des étapes de différenciation de cellules souches en cellules sécrétrices d'insuline en réponse à différentes stimulations exogène.

23. Vecteur de clonage et/ou d'expression, **caractérisé en ce qu'**il comprend un insert constitué par un polynucléotide selon l'une quelconque des revendications 8, 11 ou 12.

24. Cellule modifiée par un polynucléotide selon l'une quelconque des revendications 8, 11 ou 12 ou un vecteur selon la revendication 23.

25. Organismes non humains transgéniques, **caractérisés en ce que** tout ou partie de leurs cellules contient un polynucléotide selon l'une quelconque des revendications 8, 11 ou 12 ou un vecteur selon la revendication 23, sous une forme libre ou intégrée.

26. Utilisation d'une cellule modifiée selon la revendication 24 ou d'un organisme non humain transgénique selon la revendication 25, pour la production d'une protéine ou d'un fragment de protéine codé par un polynucléotide selon la revendication 8 ou la revendication 11, ou choisi parmi les séquences SEQ ID NO:7, SEQ ID N0:8, SEQ ID N0:9 et SEQ ID N0:10.

27. Méthode de préparation d'une protéine ou d'un fragment de protéine codé par un polynucléotide selon la revendication 8 ou la revendication 11, ou choisi parmi les séquences SEQ ID NO:7, SEQ ID NO:8, SEQ ID NO:9 et SEQ ID NO:10, **caractérisée en ce qu'**elle comprend la culture de cellules modifiées selon la revendication 24, notamment de cellules de mammifères, ou de cellules d'organismes non humains transgéniques tels que définis à la revendication 25, dans des conditions permettant l'expression de ladite protéine, et la purification de ladite protéine recombinante.

28. Utilisation d'un anticorps mono- ou polyclonal spécifique de la protéine de SEQ ID No:2, pour la détection et/ou le tri des îlots de Langerhans ou bien des cellules bêta.

29. Utilisation d'un anticorps mono- ou polyclonal spécifique de la protéine de SEQ ID No:2, pour analyser la différenciation de cellules souches en cellules d'îlot pancréatique, préférentiellement en cellules bêta.

30. Méthode de sélection des cellules bêta des îlots de Langerhans, comprenant une première étape de mise en contact des cellules d'un échantillon biologique susceptible de contenir de tels îlots et/ou cellules avec une anticorps mono-ou polyclonal spécifique de la protéine de SEQ ID No:2, une deuxième étape de mise en évidence par tout moyen approprié des cellules marquées par l'anticorps et une troisième étape d'isolement par tout moyen approprié des cellules marquées.

31. Méthode d'analyse de la différenciation de cellules souches en cellules d'îlot pancréatique ou en cellules bêta, comprenant une étape de mise en contact des cellules d'un échantillon biologique susceptible de contenir lesdites cellules souches en cours de différenciation avec un anticorps mono- ou polyclonal spécifique de la protéine de SEQ ID No:2, une deuxième étape de mise en évidence par tout moyen approprié des cellules marquées par l'anticorps et une troisième étape de visualisation par tout moyen approprié des cellules marquées.

32. Méthode selon la revendication 31 comprenant en outre une étape supplémentaire d'isolement par tout moyen approprié des cellules marquées.

33. Méthode de criblage d'un composé chimique ou biochimique pouvant interagir *in vitro* ou *in vivo* directement ou indirectement avec le polynucléotide selon l'une quelconque des revendications 8 ou 11, **caractérisée en ce qu'**elle comprend une première étape de mise en contact d'un composé chimique ou biochimique candidat avec un polynucléotide selon l'une quelconque des revendications 8 ou 11 ou une cellule selon la revendication 24 ou un organisme non-humain transgénique selon la revendication 25, ou une puce à ADN selon la revendication 20, et une deuxième étape de détection du complexe formé entre le composé chimique ou biochimique candidat et le polynucléotide ou la cellule ou l'organisme non-humain transgénique ou la puce à ADN.

34. Méthode de criblage d'un composé chimique ou biochimique pouvant moduler *in vitro* ou *in vivo* directement ou indirectement l'expression du polynucléotide selon l'une quelconque des revendications 8 ou 11, **caractérisée en ce qu'**elle comprend une première étape de mise en contact d'un composé chimique ou biochimique candidat avec un polynucléotide selon l'une quelconque des revendications 8 ou 11 ou une cellule selon la revendication 24 ou un organisme non-humain transgénique selon la revendication 25 ou une puce à ADN selon la revendication 20, et une deuxième étape de mesure par tout moyen approprié de l'expression du polynucléotide selon l'une quelconque des revendications 8 ou 11.

35. Méthode de criblage d'un composé chimique ou biochimique potentiellement utilisable pour le traitement du diabète et de l'hyperinsulinisme, **caractérisée en ce qu'**elle comprend une première étape de mise en contact d'un composé chimique ou biochimique candidat avec une protéine ou un fragment de protéine selon codé par un polynucléotide selon la revendication 8 ou la revendication 11, ou choisi parmi les séquences SEQ ID NO:7, SEQ ID NO:8, SEQ ID N0:9 et SEQ ID NO:10 ou une cellule selon la revendication 24 ou un organisme non-humain transgénique selon la revendication 25, et une deuxième étape de détection du complexe formé entre le composé chimique ou biochimique candidat et la protéine ou la cellule ou l'organisme transgénique.

36. Médicament comprenant un produit choisi parmi les polynucléotides selon l'une quelconque des revendications 8, 11 ou 12, les protéines ou fragments de protéine codés par un polynucléotide selon la revendication 8 ou la revendication 11, les polypeptides de séquences SEQ ID NO:7, SEQ ID N0:8, SEQ ID N0:9 et SEQ ID N0:10, les anticorps spécifiques de la protéine de SEQ ID No:2, les vecteurs selon la revendication 23 et les cellules modifiées selon la revendication 24.

37. Utilisation d'un produit choisi parmi : les polynucléotides selon l'une quelconque des revendications 8, 11 ou 12, les protéines ou fragments de protéine codés par un polynucléotide selon la revendication 8 ou la revendication 11, les polypeptides de séquences SEQ ID NO:7, SEQ ID NO:8, SEQ ID NO:9 et SEQ ID NO:10, les anticorps spécifiques de la protéine de SEQ ID No:2, les vecteurs selon la revendication 23 et les cellules modifiées selon la revendication 24, pour la préparation d'un médicament destiné à la prévention et/ou au traitement du diabète, particulièrement celui associé à la présence d'au moins une mutation du gène correspondant à la SEQ ID NO:1, et/ou à une expression anormale de la protéine correspondant à la SEQ ID NO:2, ou destiné à la prévention et/ou au traitement des hyperinsulinismes lorsque l'on observe une expression, une maturation ou une sécrétion anormale du gène de l'insuline ou destiné à réguler la maturation et/ou la sécrétion de l'insuline dans les cellules bêta ou dans des cellules modifiées en vue d'une sécrétion d'insuline, ou destiné à réguler les phénomènes d'apoptose des cellules bêta.

38. Utilisation d'un produit choisi parmi : les polynucléotides selon l'une quelconque des revendications 8, 11 ou 12, les protéines ou fragments de protéine codés par un polynucléotide selon la revendication 8 ou la revendication 11, les polypeptides de séquences SEQ ID NO:7, SEQ ID NO:8, SEQ ID NO:9 et SEQ ID N0:10, les anticorps spécifiques de la protéine de SEQ ID No:2, pour déterminer une variabilité allélique, une mutation, une délétion, une perte d'hétérozygotie ou toute anomalie du gène codant pour ladite protéine.

## Claims

1. Use, as a marker specific for the beta cells of the pancreatic islets of Langerhans, of at least one isolated polynucleotide or of the corresponding protein, selected from among:
the polynucleotides comprising or having one of the following sequences: (a) the sequence SEQ ID NO. 1, (b) a fragment of the sequence SEQ ID NO. 1 of at least 15 consecutive nucleotides, (c) a sequence having a percentage identity of at least 80% after optimum alignment with one of the sequences defined in (a) or (b), and (d) a sense or antisense sequence complementary to one of the sequences defined in (a), (b) or (c), and
the proteins coded by the polynucleotides as defined in (a), (b), (c) or (d) hereinbefore, comprising or having one of the following sequences:
(e) the sequence SEQ ID NO. 2, (f) a fragment of the sequence SEQ ID NO. 2 of at least 15 consecutive amino acids, (g) a sequence having a percentage identity of at least 60% after optimum alignment with one of the sequences defined in (e) or (f), preferably 80% identity, or even more preferably 90% identity.

2. Use according to claim 1, **characterised in that** said isolated polynucleotide as defined in c) is a variant polynucleotide of the sequence SEQ ID NO. 1, comprising a mutation which leads to a modification of the amino acid sequence of the protein coded by the sequence SEQ ID NO. 1.

3. Use according to claim 1, **characterised in that** said isolated polynucleotide as defined in b) or d) is selected from among the primer pair SEQ ID NO. 3 and SEQ ID NO. 27 and the primer pair SEQ ID NO. 5 and SEQ ID NO. 6.

4. Use according to claim 1, **characterised in that** said isolated polynucleotide is obtainable by amplification using the primer pair as defined in claim 3.

5. Use according to claim 1, **characterised in that** said polynucleotide as defined in d) is a small interfering RNA (siRNA) which by interaction with the mRNAs corresponding to said polynucleotide will lead to their degradation.

6. Use according to claim 1, **characterised in that** said protein as defined in (g) is a variant of the sequence SEQ ID NO. 2 having a mutation associated with diabetes or hyperinsulinism.

7. Use according to claim 1, **characterised in that** said fragment as defined in (f) has a sequence selected from among the sequences SEQ ID NO. 7, SEQ ID NO. 8, SEQ ID NO. 9 and SEQ ID NO. 10.

8. Isolated polynucleotide, **characterised in that** it can be used according to claim 1, and **in that** it comprises or has a sequence selected from among:
(a) the sequence SEQ ID NO. 1,
(b) a fragment of the sequence SEQ ID NO. 1 of at least 20 consecutive nucleotides,
(c) a sense or antisense sequence complementary to one of the sequences defined in (a) or (b),
with the exception of the fragments of at least 15 consecutive nucleotides included in the sequences having the following accession numbers in the NCBI database: no. AX526723 (SEQ ID NO. 11), no. AX526725 (SEQ ID NO. 12) and no. AX526727 (SEQ ID NO. 13), ESTs having the following accession numbers in the GenBank database: BM565129 (SEQ ID NO. 14), BM310003 (SEQ ID NO. 15), BM875526 (SEQ ID NO. 16), BG655918 (SEQ ID NO. 17), BQ417284 (SEQ ID NO. 18), BQ267316 (SEQ ID NO. 19), BU072134 (SEQ ID NO. 20), BQ267526 (SEQ ID NO. 21), BQ270198 (SEQ ID NO. 22), BU 581447 (SEQ ID NO. 23), BU070173 (SEQ ID NO. 24), BQ631692 (SEQ ID NO. 25) and BU949895 (SEQ ID NO. 26) and sequences having the following accession numbers in the NCBI database: AX526723 (SEQ ID NO. 11), AX526725 (SEQ ID NO. 12) and AX526727 (SEQ ID NO. 13).

9. Probe for detecting, identifying or titrating nucleic acids corresponding to the polynucleotides as defined in claim 1, **characterised in that** it consists of a polynucleotide according to claim 8.

10. Primer pair for amplifying nucleic acids which may be used according to claim 1, **characterised in that** it is selected from among the primer pairs as defined in claim 3.

11. Isolated polynucleotide which may be used according to claim 1, capable of being obtained by amplification using the primers according to claim 10.

12. Polynucleotide according to claim 8, **characterised in that** it is a small interfering RNA corresponding to the polynucleotide as defined in claim 1, which by interaction with the mRNAs corresponding to said polynucleotide will lead to their degradation.

13. Method of determining the transcription profile of the gene corresponding to the polynucleotide according to claim 8 or claim 11 or an alteration of said profile, in a biological sample, comprising a first step of obtaining RNAs from the biological sample, by any suitable means, a second step of contacting said RNAs with a labelled probe consisting of a polynucleotide according to any one of claims 8, 9 or 11 under appropriate hybridisation conditions between the RNAs and the probe, and a third step of revealing the hybrids formed, by any suitable means.

14. Method according to claim 13, wherein the second step is a step of retrotranscription and/or amplification of the transcripts, carried out using a primer pair according to claim 10, and the third step is a step of revealing the amplified nucleic acids, by any suitable means.

15. Method according to any one of claims 13 or 14, **characterised in that** it further comprises a step of evaluating the transcription level of the gene by comparison with a control chosen beforehand.

16. Method of detecting the gene corresponding to the polynucleotide according to claim 8 or claim 11 or allelic variants of said gene or a functional alteration of this gene, in a biological sample, comprising a first step of obtaining DNA from the biological sample by any suitable means, a second step of contacting said DNAs with a labelled probe consisting of a polynucleotide according to any one of claims 8, 9 or 11 under appropriate hybridisation conditions between the DNAs and the probe, and a third step of revealing the hybrids formed, by any suitable means.

17. Method according to claim 16, wherein the second step is an amplification step carried out using a primer pair according to claim 10 and the third step is a step of revealing the nucleic acids formed, by any suitable means.

18. Method according to any one of claims 16 or 17, **characterised in that** it further comprises a step of isolating and sequencing the nucleic acids detected.

19. Reagents kit for carrying out the methods according to any one of claims 13 to 18, comprising:
a) at least one probe according to claim 9 and/or a primer pair according to claim 10;
b) the reagents needed to carry out a hybridisation reaction between said probe and/or said primers and the nucleic acid of the biological sample;
c) the reagents needed to carry out an amplification reaction;
d) the reagents needed for detecting and/or titrating the hybrid formed between said probe and the nucleic acid of the biological sample or the amplified nucleic acids formed.

20. DNA chip comprising at least one polynucleotide according to claim 8 or claim 11.

21. Use of a polynucleotide according to claim 8 or claim 11 for preparing a DNA chip.

22. *In vitro* use of the polynucleotide according to any one of claims 8, 11 or 12, as a means of studying:
a) the overexpression of the transporter encoded by the polynucleotide according to claim 8 or claim 11 in model cell lines and the impact on insulin secretion in response to stimulation by glucose;
b) the sensitivity of the beta cells of the pancreatic islets of Langerhans to cell death (apoptosis) induced by conditions of oxidising stress or a low or high concentration of zinc;
c) steps of differentiating stem cells into insulin secreting cells in response to different exogenous stimuli.

23. Cloning and/or expression vector, **characterised in that** it comprises an insert consisting of a polynucleotide according to any one of claims 8, 11 or 12.

24. Cell modified by a polynucleotide according to any one of claims 8, 11 or 12 or a vector according to claim 23.

25. Transgenic non-human organisms, **characterised in that** all or part of their cells contains a polynucleotide according to any one of claims 8, 11 or 12 or a vector according to claim 23, in a free or integrated form.

26. Use of a modified cell according to claim 24 or a transgenic non-human organism according to claim 25, for producing a protein or a protein fragment coded by a polynucleotide according to claim 8 or claim 11, or selected from among the sequences SEQ ID NO. 7, SEQ ID NO. 8, SEQ ID NO. 9 and SEQ ID NO. 10.

27. Method of preparing a protein or a protein fragment coded by a polynucleotide according to claim 8 or claim 11, or selected from among the sequences SEQ ID NO. 7, SEQ ID NO. 8, SEQ ID NO. 9 and SEQ ID NO. 10, **characterised in that** it comprises culturing modified cells according to claim 24, notably mammalian cells, or cells of transgenic non-human organisms as defined in claim 25, under conditions that allow the expression of said protein, and the purification of said recombinant protein.

28. Use of a mono- or polyclonal antibody specific for the protein of SEQ ID NO. 2, for detecting and/or sorting islets of Langerhans or beta cells.

29. Use of a mono- or polyclonal antibody specific for the protein of SEQ ID NO. 2, for analysing the differentiation of stem cells into cells of islets of Langerhans, preferably into beta cells.

30. Method of selecting the beta cells of islets of Langerhans, comprising a first step of contacting the cells of a biological sample likely to contain such islets and/or cells with a mono- or polyclonal antibody specific for the protein of SEQ ID NO. 2, a second step of detecting the cells labelled by the antibody, by any suitable means, and a third step of isolating the labelled cells, by any suitable means.

31. Method of analysing the differentiation of stem cells into cells of islets of Langerhans or into beta cells, comprising a step of contacting the cells of a biological sample likely to contain said stem cells in the process of differentiation with a mono-or polyclonal antibody specific for the protein of SEQ ID NO. 2, a second step of detecting the cells labelled by the antibody, by any suitable means, and a third step of visualising the labelled cells, by any suitable means.

32. Method according to claim 31, further comprising a supplementary step of isolating the labelled cells by any suitable means.

33. Method of screening a chemical or biochemical compound capable of interacting *in vitro* or *in vivo* directly or indirectly with the polynucleotide according to any one of claims 8 or 11, **characterised in that** it comprises a first step of contacting a candidate chemical or biochemical compound with a polynucleotide according to any one of claims 8 or 11 or a cell according to claim 24, or a transgenic non-human organism according to claim 25, or a DNA chip according to claim 20, and a second step of detecting the complex formed between the candidate chemical or biochemical compound and the polynucleotide or cell or transgenic non-human organism or DNA chip.

34. Method of screening a chemical or biochemical compound capable of modifying the expression of the polynucleotide according to any one of claims 8 or 11, *in vitro* or *in vivo,* directly or indirectly, **characterised in that** it comprises a first step of contacting a candidate chemical or biochemical compound with a polynucleotide according to any one of claims 8 or 11 or a cell according to claim 24, or a transgenic non-human organism according to claim 25, or a DNA chip according to claim 20, and a second step of measuring the expression of the polynucleotide according to any one of claims 8 or 11 by any suitable means.

35. Method of screening a chemical or biochemical compound which is potentially useable for treating diabetes and hyperinsulinism, **characterised in that** it comprises a first step of contacting a candidate chemical or biochemical compound with a protein or a protein fragment coded by a polynucleotide according to claim 8 or claim 11, or selected from among the sequences SEQ ID NO. 7, SEQ ID NO. 8, SEQ ID NO. 9 and SEQ ID NO. 10 or a cell according to claim 24, or a transgenic non-human organism according to claim 25, and a second step of detecting the complex formed between the candidate chemical or biochemical compound and the protein or cell or transgenic organism.

36. Medicament comprising a product selected from among the polynucleotides according to any one of claims 8, 11 or 12, the proteins or protein fragments coded by a polynucleotide according to claim 8 or claim 11, the polypeptides of sequences SEQ ID NO. 7, SEQ ID NO. 8, SEQ ID NO. 9 and SEQ ID NO. 10, the antibodies specific for the protein of SEQ ID NO. 2, the vectors according to claim 23 and the modified cells according to claim 24.

37. Use of a product selected from among: the polynucleotides according to any one of claims 8, 11 or 12, the proteins or protein fragments coded by a polynucleotide according to claim 8 or claim 11, the polypeptides of sequences SEQ ID NO. 7, SEQ ID NO. 8, SEQ ID NO. 9 and SEQ ID NO. 10, the antibodies specific for the protein of SEQ ID NO. 2, the vectors according to claim 23 and the modified cells according to claim 24, for preparing a medicament intended for the prevention and/or treatment of diabetes, particularly that associated with the presence of at least one mutation of the gene corresponding to SEQ ID NO. 1, and/or with abnormal expression of the protein corresponding to SEQ ID NO. 2, or intended for the prevention and/or treatment of hyperinsulinism when there is found to be abnormal expression, maturation or secretion of the insulin gene, or intended to regulate the maturation and/or secretion of insulin in the beta cells or in the cells which are modified in terms of insulin secretion, or intended to regulate the apoptosis phenomena in the beta cells.

38. Use of a product selected from among: the polynucleotides according to any one of claims 8, 11 or 12, the proteins or protein fragments coded by a polynucleotide according to claim 8 or claim 11, the polypeptides of sequences SEQ ID NO. 7, SEQ ID NO. 8, SEQ ID NO. 9 and SEQ ID NO. 10, the antibodies specific for the protein of SEQ ID NO. 2, for determining an allelic variability, a mutation, a deletion, a loss of heterozygosity or any anomaly in the gene coding for said protein.

## Patentansprüche

1. Verwendung von mindestens einem isolierten Polynukleotid oder des entsprechenden Proteins als spezifischer Marker für Pankreas-Beta-Zellen der Langerhansschen Inseln, das wie folgt ausgewählt wird:
- aus Polynukleotiden, die eine der folgenden Sequenzen umfassen oder präsentieren: (a) die Sequenz SEQ ID NO:1, (b) ein Fragment der Sequenz SEQ ID NO: 1 aus mindestens 15 aufeinander folgenden Nukleotiden, (c) eine Sequenz, die nach optimalem Alignment zu mindestens 80 % identisch ist mit einer der unter (a) oder (b) genannten Sequenzen, und (d) eine zusätzliche Sequenz in derselben (,sense') oder in umgekehrter Richtung (,anti-sense') wie eine der unter (a), (b) oder (c) genannten Sequenzen, und
- aus den Proteinen, die von den vorstehend unter (a), (b), (c) oder (d) definierten Polynukleotiden kodiert werden und die eine der folgenden Sequenzen umfassen oder präsentieren: (e) die Sequenz SEQ ID NO:2, (f) ein Fragment der Sequenz SEQ ID NO:2 aus mindestens 15 aufeinander folgenden Aminosäuren, (g) eine Sequenz, die nach optimalem Alignment zu mindestens 60 %, vorzugsweise zu 80 % und noch idealer zu 90 % identisch ist mit einer der unter (e) oder (f) genannten Sequenzen.

2. Verwendung nach Anspruch 1, **dadurch gekennzeichnet, dass** dieses isolierte Polynukleotid nach c) eine Polynukleotid-Variante der Sequenz SEQ ID NO:1 ist, das eine Mutation enthält, die zu einer Änderung der Sequenz aus Aminosäuren des Proteins führt, das durch die Sequenz SEQ ID NO:1 kodiert ist.

3. Verwendung nach Anspruch 1, **dadurch gekennzeichnet, dass** dieses isolierte Polynukleotid nach b) oder d) aus dem Primerpaar von SEQ ID NO:3 und SEQ ID NO:27 und dem Primerpaar SEQ ID NO:5 und SEQ ID NO:6 ausgewählt wird.

4. Verwendung nach Anspruch 1, **dadurch gekennzeichnet, dass** dieses isolierte Polynukleotid durch Amplifikation mit Hilfe des in Anspruch 3 definierten Primerpaars hergestellt werden kann.

5. Verwendung nach Anspruch 1, **dadurch gekennzeichnet, dass** dieses unter (d) definierte Polynukleotid eine small interfering RNA (siRNA) ist, die durch Interaktion mit den zu diesem Polynukleotid gehörigen mRNAs zu deren Abbau führt.

6. Verwendung nach Anspruch 1, **dadurch gekennzeichnet, dass** dieses unter (g) definierte Protein eine Variante der Sequenz SEQ ID NO:2 ist, die eine mit Diabetes und Hyperinsulinämie in Zusammenhang stehende Mutation ist.

7. Verwendung nach Anspruch 1, **dadurch gekennzeichnet, dass** das unter (f) definierte Fragment eine Sequenz aufweist, die unter den Sequenzen SEQ ID NO:7, SEQ ID NO:8, SEQ ID NO:9 UND SEQ ID NO:10 ausgewählt wird.

8. Isoliertes Polynukleotid, **dadurch gekennzeichnet, dass** es gemäß Anspruch 1 verwendbar ist und eine Sequenz aufweist oder präsentiert, die ausgewählt wird aus:
(a) der Sequenz SEQ ID NO:1,
(b) einem Fragment der Sequenz SEQ ID NO:1 von mindestens 20 aufeinander folgenden Nukleotiden,
(c) einer zusätzlichen Sequenz, die in derselben (,sense') oder in der umgekehrten Richtung (,anti-sense') wie eine der Sequenzen unter (a) oder (b) verläuft;
ausgenommen sind Fragmente von mindestens 15 aufeinander folgenden Nukleotiden, die Teil der Sequenzen sind, die Zugangsnummern in der Datenbank NCBI Nr. AX526723 (SEQ ID NO: 11), Nr. AX526725 (SEQ ID NO: 12) und Nr. AX526727 (SEQ ID NO: 13) besitzen; EST mit Zugangsnummer in der Datenbank GenBank BM565129 (SEQ ID NO: 14), BM310003 (SEQ ID NO: 15), BM875526 (SEQ ID NO: 16), BG655918 (SEQ ID NO: 17), BQ417284 (SEQ ID NO: 18), BQ267316 (SEQ ID NO: 19), BU072134 (SEQ ID NO: 20), BQ267526 (SEQ ID NO: 21), BQ270198 (SEQ ID NO: 22), BU581447 (SEQ ID NO: 23), BU070173 (SEQ ID NO: 24), BQ631692 (SEQ ID NO: 25) und BU949895 (SEQ ID NO: 26) sowie Sequenzen mit Zugangsnummern in der Datenbank NCBI AX526723 (SEQ ID NO: 11) AX526725 (SEQ ID NO: 12) und AX526727 (SEQ ID NO: 13).

9. Sonde zum Aufspüren, zur Identifizierung oder Dosierung der Nukleinsäuren zu den in Anspruch 1 definierten Polynukleotiden, **dadurch gekennzeichnet, dass** sie aus einem Polynukleotid nach Anspruch 8 besteht.

10. Primerpaar zur Amplifikation von Nukleinsäuren, die gemäß Anspruch 1 verwendbar sind, **dadurch gekennzeichnet, dass** es aus den in Anspruch 3 definierten Primerpaaren ausgewählt wird.

11. Isoliertes Polynukleotid, das gemäß Anspruch 1 verwendbar ist und durch Amplifikation aus Primern nach Anspruch 10 gewonnen werden kann.

12. Polynukleotid nach Anspruch 8, **dadurch gekennzeichnet, dass** es sich um eine small interfering RNA handelt, die dem in Anspruch 1 definierten Polynukleotid zuzuordnen ist und die durch Interaktion mit den zu diesem Polynukleotid gehörigen mRNAs zu deren Abbau führt.

13. Methode zur Bestimmung des Transkriptionsprofils des zum Polynukleotid nach Anspruch 8 oder Anspruch 11 gehörigen Gens oder zur Veränderung dieses Profils in einer biologischen Probe, die einen ersten Schritt umfasst, in dem durch alle geeigneten Mittel RNAs aus der biologischen Probe erzeugt werden, einen zweiten Schritt, in dem diese RNAs unter geeigneten Hybridisierungsbedingungen zwischen den RNAs und der Sonde mit einer markierten Sonde in Kontakt gebracht werden, die aus einem Polynukleotid nach einem beliebigen der Ansprüche 8, 9 oder 11 besteht, sowie einen dritten Schritt, in dem die gebildeten Hybride durch ein beliebiges dazu geeignetes Mittel entwickelt werden.

14. Methode nach Anspruch 13, bei der der zweite Schritt eine Retrotranskription und/oder Amplifikation der Transkriptate ist, die mit Hilfe eines Primerpaares nach Anspruch 10 erfolgt, und bei der der dritte Schritt eine Schritt zur Offenlegung der amplifizierten Nukleinsäuren durch alle dazu geeigneten Mittel ist.

15. Methode nach einem beliebigen der Ansprüche 13 oder 14, **dadurch gekennzeichnet, dass** sie außerdem einen Schritt umfasst, bei dem bewertet wird, wie hoch der Transkriptionsgrad des Gens im Vergleich mit einem zuvor ausgewählten Vergleichsmuster ist.

16. Methode zum Nachweis des zum Polynukleotid gehörigen Gens nach Anspruch 8 oder Anspruch 11 oder von Allelvarianten bzw. funktional veränderten Varianten des Gens in einer biologischen Probe, die einen ersten Schritt umfasst, bei dem aus der biologischen Probe mit einem beliebigen geeigneten Mittel DNA hergestellt wird, außerdem einen zweiten Schritt, in dem diese DNA unter geeigneten Hybridisierungsbedingungen für sie und die Sonde mit einer markierten Sonde aus einem Polynukleotid gemäß einem beliebigen der Ansprüche 8, 9 oder 11 in Kontakt gebracht wird, sowie einen dritten Schritt, bei dem die gebildeten Hybride durch ein beliebiges dazu geeignetes Mittel entwickelt werden.

17. Methode nach Anspruch 16, bei der der zweite Schritt ein Amplifikationsschritt ist, der mit Hilfe eines Primerpaars nach Anspruch 10 durchgeführt wird, und der dritte Schritt ein Entwicklungsschritt, in dem die gebildeten Nukleinsäuren durch ein beliebiges geeignetes Mittel offen gelegt werden.

18. Methode nach einem beliebigen der Ansprüche 16 oder 17, **dadurch gekennzeichnet, dass** sie außerdem einen Schritt zur Isolierung und Sequenzierung der nachgewiesenen Nukleinsäuren umfasst.

19. Reagenzienkit zur Umsetzung der Methoden nach einem beliebigen der Ansprüche 13 bis 18 mit folgendem Inhalt:
(a) mindestens eine Sonde nach Anspruch 9 und/oder ein Primerpaar nach Anspruch 10
a) die für die Durchführung einer Hybridisierungsreaktion zwischen der Sonde und/oder dem genannten Primerpaar und der Nukleinsäure aus der biologischen Probe erforderlichen Reagenzien
b) die für die Durchführung einer Amplifikationsreaktion erforderlichen Reagenzien
c) die für den Nachweis und/oder die Dosierung des Hybrids aus der o. g. Sonde und der Nukleinsäure aus der biologischen Probe oder der gebildeten amplifizierten Nukleinsäuren erforderlichen Reagenzien

20. DNA-Chip, der mindestens ein Polynukleotid nach Anspruch 8 oder 11 enthält.

21. Verwendung eines Polynukleotids nach Anspruch 8 oder 11 zur Herstellung eines DNA-Chips.

22. Verwendung des Polynukleotids nach einem beliebigen der Ansprüche 8, 11 oder 12 *in vitro* als Mittel für folgende Untersuchungen:
(a) in den Modell-Zell-Linien überexprimierter vom Polynukleotid nach Anspruch 8 oder 11 kodierter Transporter und Auswirkung auf die Insulinsekretion als Antwort auf eine Stimulation durch Glucose
a) Empfindlichkeit der Beta-Zellen aus den Langerhansschen Pankreasinseln für den Zelltod (Apoptose) durch Oxidationsstress bzw. geringe oder hohe Zinkkonzentration
b) Schritte zur Differenzierung von Stammzellen in Insulin sekretierende Zellen als Antwort auf unterschiedliche exogene Stimulationen

23. Klon- oder Expressionsvektor, **dadurch gekennzeichnet, dass** er ein Inlay aus einem Polynukleotid nach einem beliebigen der Ansprüche 8, 11 oder 12 enthält.

24. Durch ein Polynukleotid nach einem beliebigen der Ansprüche 8, 11 oder 12 oder durch einen Vektor nach Anspruch 23 modifizierte Zelle.

25. Transgene nichtmenschliche Organismen, **dadurch gekennzeichnet, dass** die Gesamtheit oder ein Teil ihrer Zellen ein Polynukleotid nach einem beliebigen der Ansprüche 8, 11 oder 12 oder einen Vektor nach Anspruch 23 in freier oder integrierter Form enthält.

26. Verwendung einer modifizierten Zelle nach Anspruch 24 oder eines transgenen nichtmenschlichen Organismus nach Anspruch 25 für die Erzeugung eines Proteins oder eines Proteinfragments, das durch ein Polynukleotid nach Anspruch 8 oder 11 kodiert oder aus den Sequenzen SEQ ID NO:7, SEQ ID NO:8, SEQ ID NO: 9 und SEQ ID NO:10 ausgewählt wird.

27. Methode zur Herstellung eines Proteins oder eines Proteinfragments, das durch ein Polynukleotid nach Anspruch 8 oder 11 kodiert oder aus den Sequenzen SEQ ID NO:7, SEQ ID NO:8, SEQ ID NO: 9 und SEQ ID NO:10 ausgewählt wird, **dadurch gekennzeichnet, dass** darin die Kultur modifizierter Zellen nach Anspruch 24 inbegriffen ist, so insbesondere von Säugetierzellen oder von Zellen transgener nichtmenschlicher Organismen nach Anspruch 25, und zwar unter Voraussetzungen, die die Expression dieses Proteins und die Aufreinigung dieses rekombinierten Proteins ermöglichen.

28. Verwendung eines spezifischen mono- oder polyklonalen Antikörpers des Proteins der SEQ ID NO:2 zum Aufspüren und/oder Sortieren der Langerhansschen Inseln oder von Beta-Zellen.

29. Verwendung eines spezifischen mono- oder polyklonalen Antikörpers des Proteins der SEQ ID NO:2 zur Analyse der Differenzierung der Stammzellen in Pankreas-Inselzellen, vorzugsweise in Beta-Zellen.

30. Methode zur Selektion von Beta-Zellen der Langerhansschen Inseln, die einen ersten Schritt umfasst, bei dem die Zellen einer biologischen Probe, die höchstwahrscheinlich solche Inseln und/oder Zellen enthalten, mit einem spezifischen mono-oder polyklonalen Antikörper des Proteins der SEQ ID NO:2 in Kontakt gebracht werden, außerdem einen zweiten Schritt zum Nachweis der durch den Antikörper markierten Zellen durch geeignete Mittel und einen dritten Schritt zur Isolierung der markierten Zellen durch ein beliebiges geeignetes Mittel.

31. Methode zur Analyse der Differenzierung der Stammzellen in Pankreas-Insel-Zellen oder in Beta-Zellen, die einen Schritt umfasst, bei dem die Zellen einer biologischen Probe, die höchstwahrscheinlich solche sich differenzierenden Stammzellen enthält, mit einem spezifischen mono- oder polyklonalen Antikörper des Proteins der SEQ ID NO:2 in Kontakt gebracht werden, außerdem einen zweiten Schritt, bei dem durch den Antikörper markierte Zellen durch ein beliebiges dazu geeignetes Mittel nachgewiesen werden, sowie einen dritten Schritt, bei dem die markierten Zellen durch ein beliebiges dazu geeignetes Mittel sichtbar gemacht werden.

32. Methode nach Anspruch 31, die außerdem einen zusätzlichen Schritt zu Isolierung der markierten Zellen durch ein beliebiges dazu geeignetes Mittel umfasst.

33. Methode zur Aufbereitung einer chemischen oder biochemischen Verbindung, die *in vitro* oder *in vivo* direkt oder indirekt mit dem Polynukleotid nach einem beliebigen der Ansprüche 8 oder 11 reagieren kann, **dadurch gekennzeichnet, dass** sie einen ersten Schritt umfasst, bei dem eine viel versprechende chemische oder biochemische Verbindung mit einem Polynukleotid nach einem beliebigen der Ansprüche 8 oder 11 bzw. einer Zelle nach Anspruch 24 oder einem transgenen nichtmenschlichen Organismus nach Anspruch 25 bzw. einem ADN-Chip nach Anspruch 20 in Kontakt tritt, außerdem einen zweiten Schritt, in dem der Komplex aus der viel versprechenden chemischen oder biochemischen Verbindung und dem Polynukleotid oder der Zelle oder dem transgenen nichtmenschlichen Organismus oder dem DNA-Chip aufgespürt wird.

34. Methode zur Aufbereitung einer chemischen oder biochemischen Verbindung, die *in vitro* oder *in vivo* direkt oder indirekt die Expression des Polynukleotids nach einem beliebigen der Ansprüche 8 oder 11 modulieren kann, **dadurch gekennzeichnet, dass** sie einen ersten Schritt umfasst, bei dem die viel versprechende chemische oder biochemische Verbindung mit einem Polynukleotid nach einem beliebigen der Ansprüche 8 bis 11 oder einer Zelle nach Anspruch 24 oder einem transgenen nichtmenschlichen Organismus nach Anspruch 25 oder einem DNA-Chip nach Anspruch 20 in Kontakt gebracht wird, außerdem einen zweiten Schritt, bei dem durch ein beliebiges geeignetes Mittel die Expression des Polynukleotids nach einem beliebigen der Ansprüche 8 oder 11 gemessen wird.

35. Methode zur Aufbereitung einer chemischen oder biochemischen Verbindung, die potenziell für die Behandlung von Diabetes und Hyperinsulinämie verwendbar ist, **dadurch gekennzeichnet, dass** sie einen ersten Schritt umfasst, in dem eine viel versprechende chemische oder biochemische Verbindung mit einem Protein oder einem Proteinfragment nach [*sic!*] in Kontakt gebracht wird, das kodiert wird von einem Polynukleotid nach Anspruch 8 oder Anspruch 11 oder ausgewählt wird aus den Sequenzen SEQ ID NO:7, SEQ ID NO:8, SEQ ID NO:9 und SEQ ID NO:10, oder mit einer Zelle nach Anspruch 24 oder einem transgenen nichtmenschlichen Organismus nach Anspruch 25, außerdem einen zweiten Schritt zum Aufspüren des Komplexes aus der viel versprechenden chemischen oder biochemischen Verbindung und dem Protein bzw. der Zelle oder dem transgenen Organismus.

36. Medikament, das ein Produkt enthält, das ausgewählt wird unter den Polynukleotiden nach einem beliebigen der Ansprüche 8, 11 oder 12, den Proteinen oder Proteinfragmenten, die durch ein Polynukleotid nach Anspruch 8 oder Anspruch 11 kodiert werden, den Polypeptiden der Sequenz SEQ ID NO:7, SEQ ID NO:8, SEQ ID NO: 9 und SEQ ID NO:10, den spezifischen Antikörpern des Proteins SEQ ID NO:2, den Vektoren nach Anspruch 23 und den modifizierten Zellen nach Anspruch 24.

37. Verwendung eines Produkts, das ausgewählt wird unter den Polynukleotiden nach einem beliebigen der Ansprüche 8, 11 oder 12, den Proteinen oder Proteinfragmenten, die durch ein Polynukleotid nach Anspruch 8 oder Anspruch 11 kodiert werden, den Polypeptiden der Sequenz SEQ ID NO:7, SEQ ID NO:8, SEQ ID NO: 9 und SEQ ID NO:10, den spezifischen Antikörpern des Proteins SEQ ID NO:2, den Vektoren nach Anspruch 23 und den modifizierten Zellen nach Anspruch 24 und das zur Herstellung eines Medikaments zur Vorbeugung und/oder Behandlung von Diabetes bestimmt ist, insbesondere für den Fall mindestens einer Mutation des zur SEQ ID NO:1 gehörigen Gens und/oder einer abnormalen Expression des zur SEQ ID NO: 2 gehörigen Proteins, bzw. das zur Prävention und/oder Behandlung der Hyperinsulinämie bestimmt ist, wenn eine Expression, Reifung oder abnormale Sekretion des Geninsulins zu beobachten ist, oder das zur Regulierung der Reifung und/oder Sekretion von Insulin in den Beta-Zellen oder in für eine Insulinsekretion modifizierten Zellen bestimmt ist oder aber die Apoptosephänomene der Beta-Zellen regulieren soll.

38. Verwendung eines Produkts, das ausgewählt wird aus den Polynukleotiden nach einem beliebigen der Ansprüche 8, 11 oder 12, den Proteinen oder Proteinfragmenten, die durch ein Polynukleotid nach Anspruch 8 oder Anspruch 11 kodiert werden, Polypeptiden der Sequenzen SEQ ID NO:7, SEQ ID NO:8, SEQ ID N0: 9 und SEQ ID NO:10, den spezifischen Antikörpern des Proteins SEQ ID NO:2, um eine Allelvariabilität, eine Mutation, Deletion, einen Verlust der Heterozygotie oder alle sonstigen Anomalien des kodierenden Gens für dieses Protein nachzuweisen.


### SEQUENCE LISTING

<110> COMMISSARIAT A L'ENERGIE ATOMIQUE / UNIVERSITE JOSEPH
   FOURIER
   SEVE, Michel
   FAVIER, Alain
<120> Protéine spécifique des cellules pancréatiques bêta des îlots de Langerhans et ses applications
<130> CGA263S85
<160> 27
<170> PatentIn version 3.3
<210> 1
   <211> 1110
   <212> DNA
   <213> Homo sapiens
<400> 1
   atggagtttc ttgaaagaac gtatcttgtg aatgataaag ctgccaagat gcatgctttc      60
   acactagaaa gtgtggaact ccaacagaaa ccggtgaata aagatcagtg tcccagagag     120
   agaccagagg agctggagtc aggaggcatg taccactgcc acagtggctc caagcccaca     180
   gaaaaggggg cgaatgagta cgcctatgcc aagtggaaac tctgttctgc ttcagcaata     240
   tgcttcattt tcatgattgc agaggtcgtg ggtgggcaca ttgctgggag tcttgctgtt     300
   gtcacagatg ctgcccacct cttaattgac ctgaccagtt tcctgctcag tctcttctcc     360
   ctgtggctgt catcgaagcc tccctctaag cggctgacat ttggatggca ccgagcagag     420
   atccttggtg ccctgctctc catcctgtgc atctgggtgg tgactggcgt gctagtgtac     480
   ctggcatgtg agcgcctgct gtatcctgat taccagatcc aggcgactgt gatgatcatc     540
   gtttccagct gcgcagtggc ggccaacatt gtactaactg tggttttgca ccagagatgc     600
   cttggccaca atcacaagga agtacaagcc aatgccagcg tcagagctgc ttttgtgcat     660
   gcccttggag atctatttca gagtatcagt gtgctaatta gtgcacttat tatctacttt     720
   aagccagagt ataaaatagc cgacccaatc tgcacattca tcttttccat cctggtcttg     780
   gccagcacca tcactatctt aaaggacttc tccatcttac tcatggaagg tgtgccaaag     840
   agcctgaatt acagtggtgt gaaagagctt attttagcag tcgacggggt gctgtctgtg     900
   cacagcctgc acatctggtc tctaacaatg aatcaagtaa ttctctcagc tcatgttgct     960
   acagcagcca gccgggacag ccaagtggtt cggagagaaa ttgctaaagc ccttagcaaa    1020
   agctttacga tgcactcact caccattcag atggaatctc cagttgacca ggaccccgac    1080
   tgccttttct gtgaagaccc ctgtgactag                                    1110
<210> 2
   <211> 369
   <212> PRT
   <213> Homo sapiens
<400> 2
   Met Glu Phe Leu Glu Arg Thr Tyr Leu Val Asn Asp Lys Ala Ala Lys
   1               5                   10                  15
   Met His Ala Phe Thr Leu Glu Ser Val Glu Leu Gln Gln Lys Pro Val
               20                  25                  30
   Asn Lys Asp Gln Cys Pro Arg Glu Arg Pro Glu Glu Leu Glu Ser Gly
           35                  40                  45
   Gly Met Tyr His Cys His Ser Gly Ser Lys Pro Thr Glu Lys Gly Ala
       50                  55                  60
   Asn Glu Tyr Ala Tyr Ala Lys Trp Lys Leu Cys Ser Ala Ser Ala Ile
   65                  70                  75                  80
   Cys Phe Ile Phe Met Ile Ala Glu Val Val Gly Gly His Ile Ala Gly
                   85                  90                  95
   Ser Leu Ala Val Val Thr Asp Ala Ala His Leu Leu Ile Asp Leu Thr
               100                 105                 110
   Ser Phe Leu Leu Ser Leu Phe Ser Leu Trp Leu Ser Ser Lys Pro Pro
           115                 120                 125
   Ser Lys Arg Leu Thr Phe Gly Trp His Arg Ala Glu Ile Leu Gly Ala
       130                 135                 140
   Leu Leu Ser Ile Leu Cys Ile Trp Val Val Thr Gly Val Leu Val Tyr
   145                 150                 155                 160
   Leu Ala Cys Glu Arg Leu Leu Tyr Pro Asp Tyr Gln Ile Gln Ala Thr
                   165                 170                 175
   Val Met Ile Ile Val Ser Ser Cys Ala Val Ala Ala Asn Ile Val Leu
               180                 185                 190
   Thr Val Val Leu His Gln Arg Cys Leu Gly His Asn His Lys Glu Val
           195                 200                 205
   Gln Ala Asn Ala Ser Val Arg Ala Ala Phe Val His Ala Leu Gly Asp
       210                 215                 220
   Leu Phe Gln Ser Ile Ser Val Leu Ile Ser Ala Leu Ile Ile Tyr Phe
   225                 230                 235                 240
   Lys Pro Glu Tyr Lys Ile Ala Asp Pro Ile Cys Thr Phe Ile Phe Ser
                   245                 250                 255
   Ile Leu Val Leu Ala Ser Thr Ile Thr Ile Leu Lys Asp Phe Ser Ile
               260                 265                 270
   Leu Leu Met Glu Gly Val Pro Lys Ser Leu Asn Tyr Ser Gly Val Lys
           275                 280                 285
   Glu Leu Ile Leu Ala Val Asp Gly Val Leu Ser Val His Ser Leu His
       290                 295                 300
   Ile Trp Ser Leu Thr Met Asn Gln Val Ile Leu Ser Ala His Val Ala
   305                 310                 315                 320
   Thr Ala Ala Ser Arg Asp Ser Gln Val Val Arg Arg Glu Ile Ala Lys
                   325                 330                 335
   Ala Leu Ser Lys Ser Phe Thr Met His Ser Leu Thr Ile Gln Met Glu
               340                 345                 350
   Ser Pro Val Asp Gln Asp Pro Asp Cys Leu Phe Cys Glu Asp Pro Cys
           355                 360                 365
   Asp
<210> 3
   <211> 24
   <212> DNA
   <213> Artificial
<220>
   <223> Amorce
<400> 3
   gatgctgccc acctcttaat tgac 24
<210> 4
   <211> 24
   <212> DNA
   <213> Artificial
<220>
   <223> Amorce
<400> 4
   tcatcttttc catcctggtc ttgg 24
<210> 5
   <211> 31
   <212> DNA
   <213> Artificial
<220>
   <223> Amorce
<400> 5
   actctagaat ggagtttctt gaaagaacgt a 31
<210> 6
   <211> 28
   <212> DNA
   <213> Artificial
<220>
   <223> Amorce
<400> 6
   aatctagagt cacaggggtc ttcacaga 28
<210> 7
   <211> 16
   <212> PRT
   <213> Homo sapiens
<400> 7
   His Ile Ala Gly Ser Leu Ala Val Val Thr Asp Ala Ala His Leu Leu
   1               5                   10                  15
<210> 8
   <211> 15
   <212> PRT
   <213> Homo sapiens
<400> 8
   Cys Glu Arg Leu Leu Tyr Pro Asp Tyr Gln Ile Gln Ala Thr Val
   1               5                   10                  15
<210> 9
   <211> 16
   <212> PRT
   <213> Homo sapiens
<400> 9
   Cys Leu Gly His Asn His Lys Glu Val Gln Ala Asn Ala Ser Val Arg
   1 5 10 15
<210> 10
   <211> 13
   <212> PRT
   <213> Homo sapiens
<400> 10
   Tyr Phe Lys Pro Glu Tyr Lys Ile Ala Asp Pro Ile Cys
   1 5 10
<210> 11
   <211> 1431
   <212> DNA
   <213> unidentified
<400> 11
   cagatatcat atgaaagaca tacacacttc atgtaatgct acctgcaagt ctccctagaa     60
   aagcagtttt tgtaggtgaa aacaatgaag ccaggtaata ttgcaaggag gctgtaattt    120
   tagcagacct accaacaaca ctgatgtagg aagctcatta ttttaatttc tggagccttt    180
   taattttttc tttagaaagt gtataaataa ttgcagtgct gctttgcttc caaaactggg    240
   cagtgagttc aacaacaacg acaacaacag ccgcagctca tcctggccgt catggagttt    300
   cttgaaagaa cgtatcttgt gaatgataaa gctgccaaga tgtatgcttt cacactagaa    360
   agtgtggaac tccaacagaa accggtgaat aaagatcagt gtcccagaga gagaccagag    420
   gagctggagt caggaggcat gtaccactgc cacagtggct ccaagcccac agaaaagggg    480
   gcgaatgagt acgcctatgc caagtggaaa ctctgttctg cttcagcaat atgcttcatt    540
   ttcatgattg cagaggtcgt gggtgggcac attgctggga gtcttgctgt tgtcacagat    600
   gctgcccacc tcttaattga cctgaccagt ttcctgctca gtctcttctc cctgtggttg    660
   tcatcgaagc ctccctctaa gcggctgaca tttggatggc accgagcaga gatccttggt    720
   gccctgctct ccatcctgtg catctgggtg gtgactggcg tgctagtgta cctggcatgt    780
   gagcgcctgc tgtatcctga ttaccagatc caggcgactg tgatgatcat cgtttccagc    840
   tgcgcagtgg cggccaacat tgtactaact gtggttttgc accagagatg ccttggccac    900
   aatcacaagg aagtacaagc caatgccagc gtcagagctg cttttgtgca tgcccttgga    960
   gatctatttc agagtatcag tgtgctaatt agtgcactta ttatctactt taagccagag   1020
   tataaaatag ccgacccaat ctgcacattc atcttttcca tcctggtctt ggccagcacc   1080
   atcactatct taaaggactt ctccatctta ctcatggaag gtgtgccaaa gagcctgaat   1140
   tacagtggtg tgaaagagct tattttagca gtcgacgggg tgctgtctgt gcacagcctg   1200
   cacatctggt ctctaacaat gaatcaagta attctctcag ctcatgttgc tacagcagcc   1260
   agctgggaca gccaagtggt tcggagagaa attgctaaag cccttagcaa aagctttacg   1320
   atgcactcac tcaccattca gatggaatct ccagttgacc aggaccccga ctgccttttc   1380
   tgtgaagacc cctgtgacta gctcagtcac accgtcagtt tcccaaattt g            1431
<210> 12
   <211> 1623
   <212> DNA
   <213> unidentified
<400> 12
   cagatatcat atgaaagaca tacacacttc atgtaatgct acctgcaagt ctccctagaa 60
   aagcagtttt tgtaggtgaa aacaatgaag ccaggtaata ttgcaaggag gctgtaattt 120
   tagcagacct accaacaaca ctgatgtagg aagctcatta ttttaatttc tggagccttt 180
   taattttttc tttagaaagt gtataaataa ttgcagtgct gctttgcttc caaaactggg 240
   cagtgagttc aacaacaacg acaacaacag ccgcagctca tcctggccgt catggagttt 300
   cttgaaagaa cgtatcttgt gaatgataaa gctgccaaga tgtatgcttt cacactagaa 360
   agtgtggaac tccaacagaa accggtgaat aaagatcagt gtcccagaga gagaccagag 420
   gagctggagt caggaggcat gtaccactgc cacagtggct ccaagcccac agaaaagggg 480
   gcgaatgagt acgcctatgc caagtggaaa ctctgttctg cttcagcaat atgcttcatt 540
   ttcatgattg cagaggtcgt gggtgggcac attgctggga gtcttgctgt tgtcacagat 600
   gctgcccacc tcttaattga cctgaccagt ttcctgctca gtctcttctc cctgtggttg 660
   tcatcgaagc ctccctctaa gcggctgaca tttggatggc accgagcaca ggttttattt 720
   agcattttat ctctcatcac cctggttgtg gtgactggcg tgctagtgta cctggcatgt 780
   gagcgcctgc tgtatcctga ttaccagatc caggcgactg tgatgatcat cgtttccagc 840
   tgcgcagtgg cggccgctaa gaacattgtt ctctctttca gactaactgt ggttttgcac 900
   cagagatgcc ttggccgcaa tcacaaggaa gtacaagcca atgccagcgt cagagctgct 960
   tttgtgcatg cccttggaga tctatttcag agtatcagtg tgctaattag tgcacttatt 1020
   atctacttta agccagagta taaaatagcc gacccaatct gcacattcat cttttccatc 1080
   ctggtcttgg ccagcaccat ctctatctta aaggacttct tcttcttact catggaaggt 1140
   gtgccaaaga gcctgaatta cagtggtgtg aaagagctta ttttatcagt cgacggggtg 1200
   ctgtctgtgc acagcctgca catctggtct ctaacaatga atcaagtaat tctctcagct 1260
   catgttgcta cagcagccag ccgggacagc caagtggttc ggagagaaat tgctaaagcc 1320
   cttagcaaaa gctttacgat gcactcactc accattcaga tggaatctcc agttgaccag 1380
   gaccccgact gccttttctg tgaagacccc tgtgaactag ctcagtcaca ccgtcagttt 1440
   cccaaatttg acaggccacc ttcaaacatg ctgctatgca gtttctgcat catagaaaat 1500
   aaggaaccaa aggaagaaat tcatgtcatg gtgcaatgca cattttatct atttatttag 1560
   ttccattcac catgaaggaa gaggcactga gatccatcaa tcaattggat tatatactga 1620
   tca 1623
<210> 13
   <211> 1318
   <212> DNA
   <213> unidentified
<400> 13
   agtgagttca acaacaacga caacaacagc cgcagctcat cctggccgtc atggagtttc      60
   ttgaaagaac gtatcttgtg aatgataaag ctgccaagat gtatgctttc acactagaaa     120
   gtgtggaact ccaacagaaa ccggtgaata aagatcagtg tcccagagag agaccagagg     180
   agctggagtc aggaggcatg taccactgcc acagtggctc caagcccaca gaaaaggggg     240
   cgaatgagta cgcctatgcc aagtgggaac tctgttctgc ttcagcaata tgcttcattt     300
   tcatgattgc agaggtcgtg ggtgggcaca ttgctgggag tcttgctgtt gtcacagatg     360
   ctgcccacct cttaattgac ctgaccagtc tcctgctcag tctcttctcc ctgtggttgt     420
   catcgaagcc tccctctaag cggctgacat ttggatggca ccgagcagag atccttggtg     480
   ccctgctctc catcctgtgc atctgggtgg tgactggcgt gctagtgtac ctggcatgtg     540
   agcgcctgct gtatcctgat taccagatcc aggcgactgt gatgatcatc gtttccagct     600
   gcgcagtggc ggccaacatt gtactaactg tggttttgca ccagagatgc cttggccaca     660
   atcacaagga agtacaagcc aatgccagcg tcagagctgc ttttgtgcat gcccttggag     720
   atctatttca gagtatcagt gtgctaatta gtgcacttat tatctacttt aagccagagt     780
   ataaaatagc cgacccaatc tgcacattca tcttttccat cctggtcttg gccagcacca     840
   tcactatctt aaaggacttc tccatcttac tcatggaagg tgtgccaaag agcctgaatt     900
   acagtggtgt gaaagagctt attttagcag tcgacggggt gctgtctgtg cacagcctgc     960
   acatctggtc tctaacaatg aatcaagtaa ttctctcagc tcatgttgct acagcagcca    1020
   gccgggacag ccaagtggtt cggagagaaa ttgctaaagc ccttagcaaa agctttacga    1080
   tgcactcact caccattcag atggaatctc cagttgacca ggaccccgac tgccttttct    1140
   gtgaagaccc ctgtgactag ctcagtcaca ccgtcagttt cccaaatttg acaggccacc    1200
   ttcaaacatg ctgctatgca gtttctgcat catagaaaat aaggaaccaa aggaagaaat    1260
   tcatgtcatg gtgcaatgca tattttatct atttatttag ttccattcac catgaagg      1318
<210> 14
   <211> 592
   <212> DNA
   <213> Homo sapiens
<220>
   <221> misc_feature
   <222> (591)..(591)
   <223> n is a, c, g, or t
<400> 14
   gcacgaggct acctgcaagt ctccctagaa aagcagtttt tgtaggtgaa aacaatgaag 60
   ccaggtaata ttgcaaggag gctgtaattt tagcagacct accaacaaca ctgatgtagg 120
   aagctcatta ttttaatttc tggagccttt taattttttc tttagaaagt gtataaataa 180
   ttgcagtgct gctttgcttc caaaactggg cagtgagttc aacaacaacg acaacaacag 240
   ccgcagctca tcctggccgt catggagttt cttgaaagaa cgtatcttgt gaatgataaa 300
   gctgccaaga tgtatgcttt cacactagaa agcgtggaac tccaacagaa accggtgaat 360
   aaagatcagt gtcccagaga gagaccagag gagctggagt caggaggcat gtaccactgc 420
   cacagtggct ccaagcccac agaaaagggg gcgaatgagt acgcctatgc caagtggaaa 480
   ctctgttctg cttcagcaat atgcttcatt ttcatgattg cagaggtcgt ggaaatttag 540
   ggagtttttt cctgcttatg gacaatgctg caatgcaaaa tcttgaagaa na 592
<210> 15
   <211> 523
   <212> DNA
   <213> Homo sapiens
<400> 15
   gcacgaggct acctgcaagt ctccctagaa aagcagtttt tgtaggtgaa aacaatgaag 60
   ccaggtaata ttgcaaggag gctgtaattt tagcagacct accaacaaca ctgatgtagg 120
   aagctcatta ttttaatttc tggagccttt taattttttc tttagaaagt gtataaataa 180
   ttgcagtgct gctttgcttc caaaactggg cagtgagttc aacaacaacg acaacaacag 240
   ccgcagctca tcctggccgt catggagttt cttgaaagaa cgtatcttgt gaatgataaa 300
   gctgccaaga tgtatgcttt cacactagaa agcgtggaac tccaacagaa accggtgaat 360
   aaagatcagt gtcccagaga gagaccagag gagctggagt caggaggcat gtaccactgc 420
   cacagtggct ccaagcccac agaaaagggg gcgaatgagt acgcctatgc caagtggaaa 480
   ctctgttctg cttcagcaat atgcttcatt ttcatgattg cag 523
<210> 16
   <211> 557
   <212> DNA
   <213> Homo sapiens
<400> 16
   gcacgagctc atggaaggtg tgccaaagag cctgaattac agtggtgtga aagagcttat 60
   tttagcagtc gacggggtgc tgtctgtgca cagcctgcac atctggtctc taacaatgaa 120
   tcaagtaatt ctctcagctc atgttgctac agcagccagc cgggacagcc aagtggttcg 180
   gagagaaatt gctaaagccc ttagcaaaag ctttacgatg cactcactca ccattcagat 240
   ggaatctcca gttgaccagg accccgactg ccttttctgt gaagacccct gtgactagct 300
   cagtcacacc gtcagtttcc caaatttgac aggccacctt caaacatgct gctatgcagt 360
   ttctgcatca tagaaaataa ggaaccaaag gaagaaattc atgtcatggt gcaatgcaca 420
   ttttatctat ttatttagtt ccattcacca tgaaggaaga ggcactgaga tccatcaatc 480
   aattggatta tatactgatc agtagctgtg ttcaattgca ggaatgtgta tatagattat 540
   tcctgagtgg agccgaa 557
<210> 17
   <211> 596
   <212> DNA
   <213> Homo sapiens
<400> 17
   tttgaccccg cgtccggaaa gagcttattt tagcagtcga cggggtgctg tctgtgcaca 60
   gcctgcacat ctggtctcta acaatgaatc aagtaattct ctcagctcat gttgctacag 120
   cagccagctg ggacagccaa gtggttcgga gagaaattgc taaagccctt agcaaaagct 180
   ttacgatgca ctcactcacc attcagatgg aatctccagt tgaccaggac cccgactgcc 240
   ttttctgtga agacccctgt gactagctca gtcacaccgt cagtttccca aatttgacag 300
   gccaccttca aacatgctgc tatgcagttt ctgcatcata gaaaataagg aaccaaagga 360
   agaaattcat atcatggtgc aatgcatatt ttatctattt atttagttgc attcaccatg 420
   aaggaagagg cactgagatc catcaatcaa ttggattata tactgatcag tagctgtgtt 480
   caattgcagg aatgtgtata tagattattc ctgagtggag ccgaagtaac agctgtttgt 540
   aactatcggc aataccaaat tcatctccct tccaataatg catcttgaga acacat 596
<210> 18
   <211> 544
   <212> DNA
   <213> Homo sapiens
<400> 18
   ccgggcgaat gagtacgcct atgccaagtg gaaactctgt tctgcttcag caatatgctt 60
   cattttcatg attgcagagg tcgtgggtgg gcacattgct gggagtcttg ctgttgtcac 120
   agatgctgcc cacctcttaa ttgacctgac cagtttcctg ctcagtctct tctccctgtg 180
   gttgtcatcg aagcctccct ctaagcggct gacatttgga tggcaccgag cagagatcct 240
   tggtgccctg ctctccatcc tgtgcatctg ggtggtgact ggcgtgctag tgtacctggc 300
   atgtgagcgc ctgctgtatc ctgattacca gatccaggcg actgtgatga tcatcgtttc 360
   cagctgcgca gtggcggcca acattgtact aactgtggtt ttgcaccaga gatgccttgg 420
   ccacaatcac aaggaagtac aagccaatgc cagcgtcaga gctgcttttg tgcatgccct 480
   tggagatcta tttcagagta tcagtgtgct aattagtgca cttattatct acttttagcc 540
   agag 544
<210> 19
   <211> 555
   <212> DNA
   <213> Homo sapiens
<400> 19
   gcacgagcac ttattatcta ctttaagcca gagtataaaa tagccgaccc aatctgcaca 60
   ttcatctttt ccatcctggt cttggccagc accatcacta tcttaaagga cttctccatc 120
   ttactcatgg aaggtgtgcc aaagagcctg aattacagtg gtgtgaaaga gcttatttta 180
   gcagtcgacg gggtgctgtc tgtgcacagc ctgcacatct ggtctctaac aatgaatcaa 240
   gtaattctct cagctcatgt tgctacagca gccagccggg acagccaagt ggttcggaga 300
   gaaattgcta aagcccttag caaaagcttt acgatgcact cactcaccat tcagatggaa 360
   tctccagttg accaggaccc cgactgcctt ttctgtgaag acccctgtga ctagctcagt 420
   cacaccgtca gtttcccaaa tttgacaggc caccttcaaa catgctgcta tgcagtttct 480
   gcatcataga aaataaggaa ccaaaggaag aaattcatgt catggtgcaa tgcacatttt 540
   atctatttat ttagt 555
<210> 20
   <211> 618
   <212> DNA
   <213> Homo sapiens
<400> 20
   cccacgcgtc cgcggacgcg tgggcggacg cgtgggcgga cgcgtggggc acttattatc 60
   tactttaagc cagagtataa aatagccgac ccaatctgca cattcatctt ttccatcctg 120
   gtcttggcca gcaccatcac tatcttaaag gacttctcca tcttactcat ggaaggtgtg 180
   ccaaagagcc tgaattacag tggtgtgaaa gagcttattt tagcagtcga cggggtgctg 240
   tctgtgcaca gcctgcacat ctggtctcta acaatgaatc aagtaattct ctcagctcat 300
   gttgctacag cagccagctg ggacagccaa gtggttcgga gagaaattgc taaagccctt 360
   agcaaaagct ttacgatgca ctcactcacc attcagatgg aatctccagt tgaccaggac 420
   cccgactgcc ttttctgtga agacccctgt gactagctca gtcacaccgt cagtttccca 480
   aatttgacag gccaccttca aacatgctgc tatgcagttt ctgcatcata gaaaataagg 540
   aaccaaagga agaaattcat atcatggtgc aatgcatatt ttatctattt atttagttgc 600
   attcaccatg aaggaaga 618
<210> 21
   <211> 554
   <212> DNA
   <213> Homo sapiens
<400> 21
   gcacgagagt ctccctagaa aagcagtttt tgtaggtgaa aacaatgaag ccaggtaata 60
   ttgcaaggag gctgtaattt tagcagacct accaacaaca ctgatgtagg aagctcatta 120
   ttttaatttc tggagccttt taattttttc tttagaaagt gtataaataa ttgcagtgct 180
   gctttgcttc caaaactggg cagtgagttc aacaacaacg acaacaacag ccgcagctca 240
   tcctggccgt catggagttt cttgaaagaa cgtatcttgt gaatgataaa gctgccaaga 300
   tgtatgcttt cacactagaa agcgtggaac tccaacagaa accggtgaat aaagatcagt 360
   gtcccagaga gagaccagag gagctggagt caggaggcat gtaccactgc cacagtggct 420
   ccaagcccac agaaaagggg gcgaatgagt acgcctatgc caagtggaaa ctctgttctg 480
   cttcagcaat atgcttcatt ttcatgattg cagaggtcgt gggtgggcac attgctggga 540
   gtcttgctgt tgtc 554
<210> 22
   <211> 535
   <212> DNA
   <213> Homo sapiens
<400> 22
   gcacgaggct acctgcaagt ctccctagaa aagcagtttt tgtaggtgaa aacaatgaag 60
   ccaggtaata ttgcaaggag gctgtaattt tagcagacct accaacaaca ctgatgtagg 120
   aagctcatta ttttaatttc tggagccttt taattttttc tttagaaagt gtataaataa 180
   ttgcagtgct gctttgcttc caaaactggg cagtgagttc aacaacaacg acaacaacag 240
   ccgcagctca tcctggccgt catggagttt cttgaaagaa cgtatcttgt gaatgataaa 300
   gctgccaaga tgtatgcttt cacactagaa agcgtggaac tccaacagaa accggtgaat 360
   aaagatcagt gtcccagaga gagaccagag gagctggagt caggaggcat gtaccactgc 420
   cacagtggct ccaagcccac agaaaagggg gcgaatgagt acgcctatgc caagtggaaa 480
   ctctgttctg cttcagcaat atgcttcatt ttcatgattg cagaggtcgt ggaaa 535
<210> 23
   <211> 474
   <212> DNA
   <213> Homo sapiens
<400> 23
   tacagtggtg tgaaagagct tattttagca gtcgacgggg tgctgtctgt gcacagcctg 60
   cacatctggt ctctaacaat gaatcaagta attctctcag ctcatgttgc tacagcagcc 120
   agccgggaca gccaagtggt tcggagagaa attgctaaag cccttagcaa aagctttacg 180
   atgcactcac tcaccattca gatggaatct ccagttgacc aggaccccga ctgccttttc 240
   tgtgaagacc cctgtgacta gctcagtcac accgtcagtt tcccaaattt gacaggccac 300
   cttcaaacat gctgctatgc agtttctgca tcatagaaaa taaggaacca aaggaagaaa 360
   ttcatgtcat ggtgcaatgc atattttatc tatttattta gttgcattca ccatgaagga 420
   agaggcactg agatccatca atcaattgga ttatatactg atcagtagct gtgt 474
<210> 24
   <211> 572
   <212> DNA
   <213> Homo sapiens
<220>
   <221> misc_feature
   <222> (29)..(29)
   <223> n is a, c, g, or t
<400> 24
   gcacgaggca caattgaatc agatatcant atgaaagaca tacacacttc atgtaatgct    60
   acctgcaagt ctccctagaa aagcagtttt tgtaggtgaa aacaatgaag ccaggtaata   120
   ttgcaaggag gctgtaattt tagcagacct accaacaaca ctgatgtagg aagctcatta   180
   ttttaatttc tggagccttt taattttttc tttagaaagt gtataaataa ttgcagtgct   240
   gctttgcttc caaaactggg cagtgagttc aacaacaacg acaacaacag ccgcagctca   300
   tcctggccgt catggagttt cttgaaagaa cgtatcttgt gaatgataaa gctgccaaga   360
   tgtatgcttt cacactagaa agcgtggaac tccaacagaa accggtgaat aaagatcagt   420
   gtcccagaga gagaccagag gagctggagt caggaggcat gtaccactgc cacagtggct   480
   ccaagcccac agaaaagggg gcgaatgagt acgcctatgc caagtggaaa ctctgttctg   540
   cttcagcaat atgcttcatt ttcatgattg ca                                 572
<210> 25
   <211> 443
   <212> DNA
   <213> Homo sapiens
<400> 25
   agcagttttt gtaggtgaaa acaatgaagc caggtaatat tgcaaggagg ctgtaatttt    60
   agcagaccta ccaacaacac tgatgtagga agctcattat tttaatttct ggagcctttt   120
   aattttttct ttagaaagtg tataaataat tgcagtgctg ctttgcttcc aaaactgggc   180
   agtgagttca acaacaacga caacaacagc cgcagctcat cctggccgtc atggagtttc   240
   ttgaaagaac gtatcttgtg aatgataaag ctgccaagat gtatgctttc acactagaaa   300
   gaaggagctg caaatgaaca cttcatagca atgtggaact ccaacagaaa ccggtgaata   360
   aagatcagtg tcccagagag agaccagagg agctggagtc aggaggcatg taccactgcc   420
   acagtggctc caagcccaca gaa                                           443
<210> 26
   <211> 554
   <212> DNA
   <213> Homo sapiens
<400> 26
   agcagttttt gtaggtgaaa acaatgaagc caggtaatat tgcaaggagg ctgtaatttt 60
   agcagaccta ccaacaacac tgatgtagga agctcattat tttaatttct ggagcctttt 120
   aattttttct ttagaaagtg tataaataat tgcagtgctg ctttgcttcc aaaactgggc 180
   agtgagttca acaacaacga caacaacagc cgcagctcat cctggccgtc atggagtttc 240
   ttgaaagaac gtatcttgtg aatgataaag ctgccaagat gtatgctttc acactagaaa 300
   gtgtggaact ccaacagaaa ccggtgaata aagatcagtg tcccagagag agaccagagg 360
   agctggagtc aggaggcatg taccactgcc acagtggctc caagcccaca gaaaaggggg 420
   cgaatgagta cgcctatgcc aagtggaaac tctgttctgc ttcagcaata tgcttcattt 480
   tcatgattgc agaggtcgtg ggtgggcaca ttgctgggag tcttgctgtt gtcacagatg 540
   ctgcccacct ctta 554
<210> 27
   <211> 24
   <212> DNA
   <213> Artificial
<220>
   <223> Amorce
<400> 27
   ccaagaccag gatggaaaag atga 24